# EUROPÄISCHE PATENTSCHRIFT

(11) **EP 4 145 127 B1**
(45) Veröffentlichungstag und Bekanntmachung des Hinweises auf die Patenterteilung: **04.06.2025**
(21) Anmeldenummer: 22182732.2
(22) Anmeldetag: 04.07.2022
(51) Int. Cl.: G01N 33/00, F17D 3/12, G01L 19/08, G01N 33/22, B01F 23/10, B01F 25/313, B01F 35/21, B01F 35/214, G01L 19/00

(54) **MESSEINRICHTUNG FÜR EINE GASLEITUNG DURCHSTRÖMT VON EINEM GASSTROM, WELCHER MIT ODORIERMITTEL, BIOGAS, SYNTHESEGAS UND/ODER WASSERSTOFF ANGEREICHERT IST**
MEASURING DEVICE FOR A GAS LINE WITH A GAS FLOW ENRICHED WITH ODORANT, BIOGAS, SYNTHESIS GAS AND / OR HYDROGEN
DISPOSITIF DE MESURE POUR UNE CONDUITE DE GAZ TRAVERSÉE PAR UN FLUX DE GAZ ENRICHI EN AGENT ODORANT, EN BIOGAZ, EN GAZ DE SYNTHÈSE ET/OU EN HYDROGÈNE

(30) Priorität: 07.09.2021 DE 102021123170
(43) Veröffentlichungstag der Anmeldung: 08.03.2023
(73) Patentinhaber: Westnetz GmbH, 44139 Dortmund (DE)
(72) Erfinder: Bühner, Ronja, 48477 Hörstel (DE); Peters, Klaus, 46483 Wesel (DE)
(74) Vertreter: Bals & Vogel Patentanwälte PartGmbB

(56) Entgegenhaltungen:
- EP-A1- 3 933 250
- WO-A1-2020/104750
- CN-A- 103 684 620
- CN-U- 206 398 363
- US-A1- 2014 269 828

## Beschreibung

Die Erfindung betrifft eine Messeinrichtung für eine von einem Gasstrom, welcher mit Odoriermittel, Biogas und/oder mit Wasserstoff angereichert ist, durchströmten Gasleitung, eine Vorrichtung, welche die Messeinrichtung umfasst, sowie eine Verwendung der Messeinrichtung oder Vorrichtung für zumindest eine Messung in einer Gasleitung.

Eine Messvorrichtung mit Anströmkörper und einer drahtlosen Sendeeinheit außerhalb der Gasleitung ist aus WO 2020/104750 A1 bekannt.

Bei der leitungsgebundenen Versorgung der Allgemeinheit mit Erdgas oder Erdgasgemischen bestehend aus Erdgas-Biogas und/oder Erdgas-Wasserstoff, welches im aufbereiteten Zustand im Wesentlichen geruchslos ist, wird aus Sicherheitsgründen gefordert, dass das Erdgas einen hinreichenden Geruch aufweist, sodass etwaige Leckagen im Leitungsnetz vom Menschen subjektiv wahrnehmbar sind.

Sofern das aufbereitete Erdgas oder Erdgasgemisch keinen hinreichenden Warngeruch aufweist, ist dieses nach gesetzlichen Vorschriften zu odorieren (vgl. für Deutschland zum Beispiel das DVGW Arbeitsblatt G 280). Aus diesem Grund ist es bekannt und üblich, dem Erdgas Odoriermittel zuzugeben. Als Odoriermittel kommen üblicherweise Substanzen mit hinreichendem Warngeruch in Betracht, die dem Erdgas einen schwefelartigen Geruch verleihen. Ein weit verbreitetes Odoriermittel ist beispielsweise Tetrahydrothiophen (THT). Des Weiteren werden seit einigen Jahren auch schwefelarme und schwefelfreie Odoriermittel eingesetzt.

Üblicherweise wird Erdgas in den Gastransportnetzen (Ferntransportleitungen) odoriermittelfrei bereitgestellt. Eine Odorierung erfolgt dann von dem Gasnetzbetreiber des betreffenden Gasverteilnetzes bei der Entnahme des Gases aus dem Gastransportnetz und dessen Druckreduzierung auf den in dem Gasverteilnetz vorherrschenden Druck. Hierzu wird ein flüssiges Odoriermittel typischerweise über eine Odorieranlage in einer örtlichen Gas-Druckregel- und Messanlage (GDRM-Anlage) zugegeben. Die Zugabe erfolgt in Abhängigkeit der Größe eines normierten Gasvolumenstroms berechnet aus den Daten von Gaszählern sowie der Gastemperatur und dem Gasdruck. Eine entsprechende Menge an Odoriermittel wird mittels einer impulsgesteuerten Dosierpumpe einer Einspeiseeinrichtung (auch als Odorierdüse oder Impfdüse bezeichnet) zugeführt, die häufig als in die Leitung eingesetzte Tauchrohr-förmige Odoriermitteldüse mit einem Verdunstungskörper ausgeführt ist, auf den das der Odoriermitteldüse zugeführte Odoriermittel in flüssiger Form aufgebracht wird.

Typischerweise erfolgt die Zugabe des Odoriermittels mengenproportional zur durchflossenen Gasmenge. Das Odoriermittel THT wird beispielsweise in Deutschland mit einem Wert von mindestens 10 mg/m3 Gasvolumen dem Gasstrom zugeführt. Für andere Odoriermittel gelten andere, zum Teil geringere Grenzwerte. Dabei ist darauf zu achten, dass ein vorbestimmter Grenzwert, beispielsweise von 10 mg/m3 bei THT, während der gesamten Verteilung des Gases nicht unterschritten wird. Das Odoriermittel wird typischerweise intermittierend mit der in die Gasleitung eingesetzten Einspeiseeinrichtung zugeführt, wobei ein Anströmkörper der Einspeiseeinrichtung von dem die Gasleitung durchströmenden Erdgas umströmt wird. Dabei verdampft das flüssige Odoriermittel von dem Verdunstungskörper und wird von dem Erdgas aufgenommen.

Aufgabe der Erfindung ist es, eine die Einspeiseeinrichtung auf vorteilhafte Art und Weise ergänzende Messeinrichtung bereitzustellen, welche auf einfache und kostengünstige Art und Weise im Zusammenhang mit der Einspeisung des Odoriermittels in den Gasstrom stehende Messungen ermöglicht.

Die voranstehende Aufgabe wird durch die Gegenstände der Patentansprüche gelöst. Insbesondere wird die Aufgabe durch eine Messeinrichtung gemäß Anspruch 1, eine Vorrichtung gemäß Anspruch 13 und eine Verwendung der Messeinrichtung oder Vorrichtung gemäß Anspruch 15 gelöst. Weitere Vorteile und Details der Erfindung ergeben sich aus den Unteransprüchen, der Beschreibung und den Zeichnungen. Dabei gelten Merkmale und Details, die im Zusammenhang mit der erfindungsgemäßen Messeinrichtung offenbart sind, selbstverständlich auch im Zusammenhang mit der erfindungsgemäßen Vorrichtung und der erfindungsgemäßen Verwendung sowie jeweils umgekehrt, sodass bezüglich der Offenbarung zu den einzelnen Erfindungsaspekten stets wechselseitig Bezug genommen wird beziehungsweise werden kann.

Gemäß einem ersten Aspekt der Erfindung wird die eingangs gestellte Aufgabe gelöst durch eine Messeinrichtung für eine von einem Gasstrom, welcher mit Odoriermittel, Biogas, Synthesegas und/oder Wasserstoff angereichert oder vermischt ist, durchströmten Gasleitung. Die Messeinrichtung weist einen Anstromkörper zur Positionierung in dem Gasstrom in der Gasleitung auf. Der Anstromkörper ist mit zumindest einem Sensor ausgestattet. Die Messeinrichtung umfasst eine drahtlose Sendeeinheit, welche zum drahtlosen Senden der Messdaten des zumindest einen Sensors an eine außerhalb der Gasleitung befindliche drahtlose Empfangseinheit eingerichtet ist. Die Messeinrichtung ist dazu eingerichtet, in eine Wandöffnung eines Gasleitungsabschnitts der Gasleitung eingesetzt zu werden, sodass sich der Anstromkörper in den Gasleitungsabschnitt erstreckt.

Die erfindungsgemäße Messeinrichtung erlaubt mittels des zumindest einen Sensors, welcher sich im oder am Anstromkörper befindet, eine zuverlässige Messung im Zusammenhang mit der Einspeisung des Odoriermittels in den Gasstrom durch eine Einspeiseeinrichtung.

Die drahtlose Übertragung der Messdaten des zumindest einen Sensors ermöglicht dabei, dass die Messeinrichtung vollständig abgeschlossen bzw. gekapselt sein kann. Die Messeinrichtung kann in so einem Falle auch als eine Messkapsel bezeichnet werden. Durch den Verzicht auf eine drahtgebundene Übertragung von Messdaten wird ein komplexer und teurer mechanischer Aufbau der Messeinrichtung vermieden. Denn durch den Verzicht auf Kabel für eine drahtgebundene Übertragung wird nicht nur das Risiko einer Gasleckage im Bereich der Drahtführung von der Messeinrichtung nach außerhalb der Gasleitung reduziert, auch eine kostenintensive Abdichtung der Drahtführung wird vermieden. Auch kann die Messeinrichtung austauschbar ausgestaltet sein, wie später näher erläutert wird. Ein Austausch der Messeinrichtung ohne Verdrahtung für eine drahtgebundene Übertragung von Messdaten und mit der erfindungsgemäß vorgeschlagenen drahtlosen Übertragung gestaltet sich deutlich einfacher.

Die Messeinrichtung ist dazu eingerichtet, in die Wandöffnung des Gasleitungsabschnitts der Gasleitung eingesetzt zu werden, so dass sich der Anströmkörper in den Gasleitungsabschnitt erstreckt. Zu diesem Zweck kann die Messeinrichtung Befestigungsmittel zur Befestigung der Einspeiseeinrichtung an der Wandöffnung aufweisen. Wenn die Messeinrichtung eine Odoriermitteldüse aufweist, wie später näher beschrieben wird, können sich die Befestigungsmittel insbesondere an der Odoriermitteldüse befinden.

Die Messeinrichtung umfasst eine drahtlose Übertragungseinheit bestehend aus der drahtlosen Sendeeinheit und optional der drahtlosen Empfangseinheit. Während sich die drahtlose Sendeeinheit an oder in dem Anstromkörper und damit innerhalb des Gasstroms befindet, ist die drahtlose Empfangseinheit außerhalb der Gasleitung und damit außerhalb vom Gasstrom angeordnet oder anordenbar. Die Sendeeinheit fungiert damit als ein Innenbauteil, während die Empfangseinheit als ein Außenbauteil fungiert. Innen und außen bezieht sich dabei jeweils auf die Gasleitung. Anders als die Sendeeinheit kann die Empfangseinheit damit innerhalb der drahtlosen Reichweite zwischen Sendeeinheit und Empfangseinheit an einer beliebigen Position außerhalb des Anstromkörpers und der Gasleitung angeordnet werden. Während die Messeinrichtung mit dem Anstromkörper und der Sendeeinheit als eine gemeinsame Komponente ausgebildet ist, kann die Empfangseinheit damit eine von dieser Komponente physisch losgelöste Komponente der Messeinrichtung sein. Möglich ist ferner, dass die Empfangseinheit mit einer Datenfernübertragungseinheit verbunden wird, um die Messdaten zum Zwecke ihrer Auswertung an einen entfernten Rechner zu übertragen.

Der Sensor kann durch entsprechende Positionierung in oder an dem Anstromkörper unmittelbar in dem Gasstrom oder neben dem Gasstrom angeordnet werden, um so direkt den Gasstrom zu messen oder eine Messung neben dem Gasstrom vorzunehmen. Möglich ist auch eine Kombination aus einem oder mehr Sensoren im Gasstrom und einem oder mehr Sensoren neben dem Gasstrom durch entsprechende Positionierung in oder an dem Anstromkörper. Die Anordnung des Sensors oder der Sensoren im oder neben dem Gasstrom kann von der zu messenden Messgröße abhängig gemacht werden. Beispielsweise können Messgrößen bzgl. der Gaszusammensetzung durch Messung einzelner Gaskomponenten einzelner Gasbestandteile wie z.B. Odoriermittel, Methan, sonstige Kohlenwasserstoffe, CO2, Schwefel oder Wasserstoff vorteilhafterweise direkt im Gasstrom gemessen werden.

Es kann vorgesehen sein, dass der zumindest eine Sensor zum Messen eines Drucks, einer Temperatur, einer Gaszusammensetzung und/oder eines Odoriermittelgehalts in dem Gasstrom eingerichtet ist. Insoweit kann es sich bei dem zumindest einen Sensor um beispielsweise einen Drucksensor, Temperatursensor, Infrarot-spektroskopiesensor, Wärmeleitfähigkeitssensor, optischen Sensor oder ähnlich handeln. Möglich ist auch der Einsatz verschiedener Sensoren bzw. Sensortypen in der Messeinrichtung. Auch der Einsatz von Kombinationssensoren, die verschiedene der vorgenannten Messgrößen des Gasstromes messen können, ist möglich.

Auch kann vorgesehen sein, dass der zumindest eine Sensor zumindest eine autarke Energieversorgungseinheit aufweist. Bei mehreren Sensoren kann jeder Sensor jeweils eine eigene autarke Energieversorgungseinheit aufweisen. Möglich ist aber auch, dass mehreren Sensoren eine gemeinsame autarke Energieversorgungseinheit zugeordnet ist. Unter einer autarken Energieversorgungseinheit wird dabei eine solche Energieversorgungseinheit verstanden, die den oder die Sensoren mit einem Strom bzw. Energie versorgen kann, der nicht von außerhalb der Gasleitung, sondern von innerhalb der Gasleitung bereitgestellt wird. Die autarke Energieversorgungseinheit sorgt, wie schon die Vermeidung der drahtgebundenen Übertragung der Messdaten, für einen einfacheren und kostengünstigeren Aufbau der Messeinrichtung, wobei zudem ein Leckagerisiko der Messeinrichtung erheblich reduziert werden kann, weil eine Gasdichtheit einfach sichergestellt werden kann.

Dabei kann vorgesehen sein, dass die zumindest eine autarke Energieversorgungseinheit als eine Batterie ausgebildet ist. Dies stellt eine besonders kostengünstige Ausführungsvariante der autarken Energieversorgungseinheit dar. Die gespeicherte Energie der Batterie ist begrenzt, was nach einer bestimmten Nutzungsdauer einen Wechsel der Batterie erforderlich macht. Die Messeinrichtung ist aber, wie bereits erwähnt, vorzugsweise austauschbar ausgestaltet, sodass sich die Messeinrichtung einfach austauschen lässt oder zumindest aus der Gasleitung entnehmen lässt, um die Batterie oder die Batterien zu tauschen.

Alternativ oder zusätzlich kann vorgesehen sein, dass die zumindest eine autarke Energieversorgungseinheit als eine Generatoreinheit ausgebildet ist, welche durch den Gasstrom angetrieben wird. Bei der Generatoreinheit kann es sich beispielsweise um einen Vortex-Generator handeln, der am oder im Anstromkörper angeordnet ist. Der Vortex-Generator kann Schwingungen des Anstromkörpers in elektrische Energie umwandeln, wozu der Anstromkörper derart ausgeführt sein kann, dass der Gasstrom den Anstromkörper zum Schwingen anregt. Möglich ist auch, dass die Generatoreinheit als ein Strömungsgenerator ausgebildet ist, der durch den Gasstrom angetrieben elektrische Energie erzeugen kann. Dabei kann die Generatoreinheit unmittelbar zum Versorgen des Sensors oder der Sensoren eingesetzt werden. Bevorzugt wird jedoch eine Kombination aus Generatoreinheit oder Generatoreinheiten mit Batterie oder Batterien als autarke Energieversorgungseinheiten bereitgestellt. Mittels der Generatoreinheit oder den Generatoreinheiten kann dann die Batterie wiederaufgeladen werden. So kann sichergestellt werden, dass der zumindest eine Sensor stets innerhalb der Rohrleitung ohne Zuleitungen von außen mit Energie versorgt werden kann. Ein Entnehmen der Messeinrichtung aufgrund einer entleerten Energieversorungsbatterie aus der Gasleitung ist nicht erforderlich. Die Energieversorgung des Sensors kann dadurch autark ausgeführt werden.

Grundsätzlich kann die drahtlose Übertragung der Messdaten mittels Funkübertragung erfolgen. Dies limitiert jedoch die Materialwahl der Messeinrichtung, weil die Messdaten mittels Funks durch das Material der Messeinrichtung, insbesondere eines äußeren Gehäuses bzw. einer Einsatzeinheit der Messeinrichtung, oder der Gasleitung gesendet werden müssten. Hierfür könnte die Messeinrichtung (ggf. auch die Gasleitung) zwar aus einem funkdurchlässigen Material, etwa Kunststoff, gefertigt werden. Dies ist jedoch eine wenig stabile Lösung, sodass eine Konstruktion aus einem Metall, insbesondere Stahl, bevorzugt ist. Dabei wird jedoch durch die Messeinrichtung bzw. ihr Gehäuse oder ihre Einsatzeinheit ein farradayscher Käfig gebildet, der eine Funkverbindung verhindert.

Daher kann vorgesehen sein, dass die drahtlose Sendeeinheit zum drahtlosen Senden der Messdaten mittels Ultraschalls eingerichtet ist. Mit anderen Worten kann die drahtlose Sendeeinheit als eine Ultraschall-Sendeeinheit ausgebildet sein. Damit wird die Problematik der nicht aufbaubaren Funkverbindung umgangen und eine sichere und kostengünstige drahtlose Übertragung der Messdaten bei gleichzeitig möglichst stabiler Ausgestaltung der Messeinrichtung bzw. ihres Gehäuses oder ihrer Einsatzeinheit aus Metall gewährleistet.

Alternativ oder zusätzlich kann vorgesehen sein, dass die drahtlose Sendeeinheit zum drahtlosen Senden der Messdaten mittels Induktion eingerichtet ist. Mit anderen Worten kann die drahtlose Sendeeinheit als eine Induktions-Sendeeinheit ausgebildet sein. Damit wird die Problematik der nicht aufbaubaren Funkverbindung umgangen und eine sichere und kostengünstige drahtlose Übertragung der Messdaten bei gleichzeitig möglichst stabiler Ausgestaltung der Messeinrichtung bzw. ihres Gehäuses oder ihrer Einsatzeinheit gewährleistet.

Alternativ oder zusätzlich kann vorgesehen sein, dass die drahtlose Sendeeinheit zum drahtlosen Senden der Messdaten mittels Lichts eingerichtet ist. Mit anderen Worten kann die drahtlose Sendeeinheit als eine Licht-Sendeeinheit ausgebildet sein. Für die Übertragung des Lichts zwischen der Sendeinheit und der Empfangseinheit ist ein Lichtkanal erforderlich. Dieser kann durch die Messeinrichtung, insbesondere ein sie umfassendes Gehäuse oder eine Einsatzeinheit, und/oder die Gasleitung hindurch ausgebildet werden. Dazu können entsprechende Bohrungen in das Material von Messeinrichtung bzw. Gehäuse oder Einsatzeinheit eingebracht sein, die mit lichtdurchlässigen Material, insbesondere Glas- oder Plexiglaslinsen, verschlossen und abgedichtet werden. Das durchsichtige Material kann elastisch mit den Bohrungen verklebt sein. Glas- oder Plexiglaslinsen, die optisch geformt sein können, ermöglichen das Lenken oder Bündeln des Lichts auf die entsprechende Licht-Empfangseinheit. Damit wird die Problematik der nicht aufbaubaren Funkverbindung umgangen und eine sichere und kostengünstige drahtlose Übertragung der Messdaten bei gleichzeitig möglichst stabiler Ausgestaltung der Messeinrichtung bzw. ihres Gehäuses oder ihrer Einsatzeinheit gewährleistet.

Grundsätzlich kann der Anstromkörper einen Gitterkörper aufweisen oder durch einen Gitterkörper gebildet werden. Der Gitterkörper kann insbesondere zylinderförmig ausgebildet sein. Der Gitterkörper kann insbesondere in Form eines Drahtkäfigs ausgebildet sein, insbesondere aus einem Drahtgitter. Mit einem Gitterkörper lassen sich die gewünschten mechanischen Eigenschaften des Anströmkörpers erreichen, um dem Gasstrom in der Gasleitung zu widerstehen. Der Gitterkörper ermöglicht zudem eine einfache Anordnung des zumindest einen Sensors daran oder darin.

Es kann insbesondere vorgesehen sein, dass die Messeinrichtung eine Einspeiseeinrichtung zum Einspeisen des flüssigen Odoriermittels in den Gasstrom aufweist, wobei die Einspeiseeinrichtung eine Odoriermitteldüse und einen Verdunstungskörper in dem Anstromkörper aufweist, und wobei die Odoriermitteldüse zur Beaufschlagung des Verdunstungskörpers mit dem flüssigen Odoriermittel ausgebildet ist. Mit anderen Worten wird eine kombinierte Einrichtung bereitgestellt, welche die Messeinrichtung mit der Einspeiseeinrichtung kombiniert. Diese Einrichtung wird im Folgenden weiter als Messeinrichtung bezeichnet, welche über die Einspeiseeinrichtung bzw. deren Mittel, insbesondere Odoriermitteldüse und Verdunstungskörper, verfügt. Dadurch kann eine einzige Einrichtung bereitgestellt werden, die ein Odorieren und Messen ermöglicht.

Eine solche Einspeiseeinrichtung erlaubt eine zuverlässige Odorierung eines Gasstroms in einer Gasleitung. Das im Betrieb von der Odoriermitteldüse auf den Verdunstungskörper gelangende Odoriermittel wird bei Um- oder Durchströmen des Verdunstungskörpers mit dem in der Gasleitung strömenden Gasstrom verdampft und vermischt sich mit dem Gasstrom, so dass dieser odoriert wird.

Die Odoriermitteldüse ist zur Beaufschlagung des Verdunstungskörpers mit flüssigem Odoriermittel ausgebildet. Zu diesem Zweck weist die Odoriermitteldüse insbesondere eine Öffnung auf, aus der im Betrieb flüssiges Odoriermittel auf den Verdunstungskörper gelangen kann. Weiterhin weist die Odoriermitteldüse vorzugsweise einen Anschluss zum Anschließen der Odoriermitteldüse an eine Odoriermittel-Versorgung auf. Unter Odoriermitteln werden vorliegend insbesondere Odoriermittel nach DIN EN ISO 13 734 verstanden.

Die Odoriermitteldüse kann insbesondere in Form einer Tauchhülse ausgebildet sein, die zum Einführen in eine Wandöffnung eines Gasleitungsabschnitts ausgebildet ist und zu diesem Zweck vorzugsweise Befestigungsmittel, beispielsweise ein Außengewinde, zur gasdichten Befestigung am Gasleitungsabschnitt aufweisen.

Die Odoriermitteldüse und der Anströmkörper weisen vorzugsweise zueinander komplementäre Befestigungsmittel auf, so dass der Anströmkörper an der Odoriermitteldüse befestigt werden kann. Auf diese Weise können der Anströmkörper oder der Verdunstungskörper ausgetauscht werden. Beispielsweise kann der Anströmkörper ein Außengewinde, insbesondere ein ISO-Gewinde, und die Odoriermitteldüse ein dazu passendes Innengewinde aufweisen oder umgekehrt.

Vorgesehen sein kann dabei, dass der Anstromkörper einen Gitterkörper aufweist, in dem der Verdunstungskörper angeordnet ist. Bei diesem Gitterkörper kann es sich um den bereits beschriebenen Gitterkörper handeln. Ferner kann vorgesehen sein, dass der Gitterkörper an dem von der Odoriermitteldüse entfernten Ende des Gitterkörpers ein becherförmiges Bodenteil aufweist, in den der Verdunstungskörper vorzugsweise eintaucht, wobei der zumindest eine Sensor in dem becherförmigen Bodenteil angeordnet ist. Durch das becherförmige Bodenteil kann überschüssiges Odoriermittel aufgefangen werden, so dass das Odoriermittel nicht in die Gasleitung tropft und diese verunreinigt. Vorzugsweise taucht der Verdunstungskörper in das becherförmige Bodenteil ein, insbesondere bis zum Grund des becherförmigen Bodenteils. Auf diese Weise kann das im becherförmigen Bodenteil angesammelte Odoriermittel über die Kapillarwirkung der Poren des Verdunstungskörpers der Verdampfung zugeführt werden. Durch den Sensor in dem becherförmigen Bodenteil kann ermittelt werden, wie viel Odoriermittel sich in dem Bodenteil über eine bestimmte Zeitdauer ansammelt. Insoweit kann der Sensor als ein Füllstandsensor des Bodenteils ausgebildet sein, welche einen Füllstand des Bodenteils mit Odoriermittel ermittelt. Über den Füllstand im becherförmigen Bodenteil kann auf die Verdunstungsrate der Odoriermitteldüse zurückgeschlossen werden. Mit anderen Worten kann es sich bei dem einen Sensor oder einem Sensor um einen Flüssigkeitssensor zur Detektion von Flüssigkeit im becherförmigen Bodenteil handeln. Das Vorhandensein von Flüssigkeit im becherförmigen Bodenteil kann auf eine schlechte Verdunstungsleistung oder auf eine zu große Zufuhr von Odoriermittel hinweisen. Neben dem Füllstand- oder Flüssigkeitssensor können ein oder mehrere weitere Sensoren an dem Gitterkörper oberhalb des Bodenteils angeordnet sein, um weitere Messgrößen, insbesondere die bereits genannten Messgrößen, zu erfassen. Dadurch kann der Gitterkörper insgesamt als eine Messsonde mit vielerlei Sensoren zum Erfassen unterschiedlicher Messgrößen, sowohl des Gases im Gasstrom, als auch des Odoriermittels im Bodenteil, ausgebildet werden.

Vorzugsweise weist der Anströmkörper eine Länge von 100 - 400 mm und/oder einen Durchmesser von 0,5 - 1 Zoll auf. Während im Stand der Technik größere Anströmkörper oder mit sehr geringer innenliegender Verdunstungsoberfläche üblich waren, wurde vorliegend festgestellt, dass bereits mit einer deutlich kleineren Geometrie des Anströmkörpers eine ausreichende Odorierung eines Gasstroms erreicht werden kann. Hierdurch wird die Handhabung der Einspeiseeinrichtung wesentlich vereinfacht, wodurch insbesondere ein schneller und damit kostenreduzierter Wechsel der Einspeiseeinrichtung ermöglicht wird, insbesondere bei Verwendung der zuvor beschriebenen Vorrichtung. Zudem wird durch den kleineren Anströmkörper das Risiko eines ungewollten Austretens oder Vergießens von Odoriermittel reduziert, insbesondere beim Wechsel der Einspeiseeinrichtung. Die kleinere Größe ermöglicht zudem das Einbringen von Odoriermittel in gasdurchströmte Gasleitungen mit sehr unterschiedlichen Nennweiten, insbesondere von 50 - 1400 mm.

Bei einer möglichen Ausführungsform des Verdunstungskörpers umfasst der Verdunstungskörper einen offenzelligen Schaum, insbesondere einen Metall- oder Keramikschaum, oder besteht zumindest teilweise, vorzugsweise vollständig, daraus. Durch die Verwendung eines offenzelligen Schaums für den Verdunstungskörper weist dieser eine sehr große Oberfläche auf, wodurch die Verdunstungsleistung stark erhöht wird und damit kleinere Anströmkörper ermöglicht werden. Bei dem Metall- oder Keramikschaum kann es sich insbesondere um einen Aluminiumschaum, um einen Nickel-Chrom-Schaum (z. B. NC2733, NC1723, NC0610, NC1116), um einen Nickelschaum (z. B. NI 1116), um einen Aluminiumoxidschaum (z. B. A1203 30, A1203 20, A1203 40), um einen Siliziumcarbidschaum (z. B. SiC 20) oder um Kombinationen daraus handeln. Derartige Schäume sind zum Beispiel über Recemat BV (Dodewaards, NL) oder Porosium GmbH (Coburg, DE) erhältlich. Oxidische Schäume, wie zum Beispiel Aluminiumoxidschaum, sind chemisch sehr stabil gegenüber den in der Praxis verwendeten Odoriermitteln und daher bevorzugt.

Bei einer weiteren möglichen Ausführungsform weist der offenzellige Schaum des Verdunstungskörpers zumindest abschnittsweise eine Porendichte von 10 - 60 ppi auf. Auf diese Weise wird die Verdunstungsleistung für die handelsüblichen Odorierstoffe, insbesondere nach DIN EN ISO13 734, und typische Gasströme in Gasleitungen optimiert, wodurch eine weitere Reduzierung der Anströmkörpergröße möglich ist.

Bei einer weiteren möglichen Ausführungsform weist der Verdunstungskörper mehrere Abschnitte auf, wobei die einzelnen Abschnitte unterschiedliche Porendichten und/oder unterschiedliche mittlere Porengrößen aufweisen. Der Verdunstungskörper kann zu diesem Zweck beispielsweise mehrere übereinander gestapelte Elemente mit jeweils verschiedenen Porendichten und/oder jeweils verschiedenen mittleren Porengrößen aufweisen. Alternativ kann auch ein monolithischer Verdunstungskörper mit insbesondere abschnittsweise unterschiedlichen Porendichten und/oder mittleren Porengrößen verwendet werden, beispielsweise durch Verwendung eines im 3D-Druck hergestellten Verdunstungskörpers.

Die Gasgeschwindigkeit eines Gasstroms in einer Gasleitung ist über den Querschnitt der Gasleitung nicht konstant, sondern weist eine Geschwindigkeitsverteilung auf, wobei sich die Geschwindigkeit am Rand der Gasleitung typischerweise von der Geschwindigkeit in der Mitte der Gasleitung unterscheidet. Durch das Vorsehen mehrerer Abschnitte mit unterschiedlicher Porendichte kann die jeweilige Porendichte an die Gasgeschwindigkeit der jeweiligen Position in Bezug auf den Gasleitungsquerschnitt angepasst werden. Entsprechend kann durch das Vorsehen mehrerer Abschnitte mit unterschiedlichen mittleren Porengrößen die jeweilige mittlere Porengröße an die Gasgeschwindigkeit der jeweiligen Position in Bezug auf den Gasleitungsquerschnitt angepasst werden.

Bei einer weiteren möglichen Ausführungsform nimmt die Porendichte und/oder die mittlere Porengröße der einzelnen Abschnitte des Verdunstungskörpers von dem von der Odoriermitteldüse entfernten Ende des Verdunstungskörpers in Richtung der Odoriermitteldüse zumindest abschnittsweise zu oder ab. Durch einen solchen über Abschnitte mit verschiedenen Porendichten bzw. mittleren Porengrößen erreichten Gradienten kann der Verdunstungskörper zum Beispiel an die langsameren Gasgeschwindigkeiten zum Rand der Gasleitung angepasst werden.

Es ist insbesondere möglich, den Verlauf der Porendichte und/oder der mittleren Porengröße über den Verdunstungskörper an die im Sommer oder im Winter in einer Gasleitung im Mittel herrschenden Gasgeschwindigkeitsverteilungen anzupassen. Es wurde festgestellt, dass sich die Gasgeschwindigkeitsverteilungen zwischen Sommer und Winter stark unterscheiden können. So kann es im Winter durch die größeren zu transportierenden Gasmengen und die damit einhergehenden größeren Gasgeschwindigkeiten zu mehr Turbulenzen in der Gasleitung kommen, wodurch sich gegenüber dem Sommer eine veränderte Geschwindigkeitsverteilung in der Gasleitung einstellt, insbesondere am Rand der Gasleitung.

Da die hier beschriebene Einspeiseeinrichtung einen schnellen Wechsel der Einspeiseeinrichtung ermöglicht, ist es wirtschaftlich möglich und sinnvoll, zum Beispiel zweimal im Jahr zwischen einer für den Sommer und einer für den Winter optimierten Einspeiseeinrichtung zu wechseln.

Bei einer weiteren möglichen Ausführungsform ist der Verdunstungskörper im 3D-Druck hergestellt. Insbesondere kann beim 3D-Druck die offenzellige Schaumstruktur des Verdunstungskörpers direkt gedruckt werden. Der 3D-Druck ermöglicht weiterhin einen Porendichtegradienten und/oder einen Porengrößengradienten, insbesondere wie zuvor beschrieben, in einem monolithischen Verdunstungskörper. Darüber hinaus ermöglicht der 3D-Druck auch die Herstellung eines selbst tragenden Verdunstungskörpers. Auf diese Weise kann auf einen Gitterkörper verzichtet werden. Alternativ kann der Gitterkörper auch direkt mitgedruckt werden. Vorzugsweise weist der Verdunstungskörper ein 3D-gedrucktes Gewinde, vorzugsweise ISO-Gewinde auf, um den Verdunstungskörper mit der Odoriermitteldüse zu verbinden. Zur Herstellung des Verdunstungskörpers im 3D-Druck kommen Kunststoffe, Metalle oder andere 3D-druckbare Werkstoffe in Betracht, soweit diese gegenüber den zu verwendenden Odoriermitteln oder in den Gasleitungen geleiteten Gasen resistent sind.

Bei einer weiteren möglichen Ausführungsform ist der Gitterkörper mit stabilisierenden Verstrebungen ausgestattet. Auf diese Weise kann der Gitterkörper stabilisiert werden, so dass dieser dem Gasstrom in der Gasleitung mechanisch widerstehen kann. Insbesondere können auf diese Weise Vibrationen reduziert oder erhöht werden, die durch das umströmende Gas entstehen und zu einer mechanischen Belastung oder zu einer erhöhten Energiegewinnung mittels eines Vortex-Generators führen. Darüber hinaus können durch das Vorsehen stabilisierender Verstrebungen andere Teile des Gitterkörpers dünner ausgestaltet werden, beispielsweise dünnere Drähte verwendet werden, wodurch eine größere Maschenweite und/oder ein höherer Maschenanteil und damit eine bessere Anströmbarkeit des im Gitterkörper angeordneten Verdunstungskörpers erreicht werden. Auf diese Weise kann die Verdunstungsleistung erhöht werden.

Bei einer weiteren möglichen Ausführungsform sind als stabilisierende Verstrebungen mehrere dickere Längsdrähte vorgesehen. Beispielsweise kann der Gitterkörper ein Drahtgitter aufweisen, bei dem einige Längsdrähte eine größere Dicke aufweisen. Weist das Drahtgitter zum Beispiel 50 oder 100 Längsdrähte auf, so können zum Beispiel vier Längsdrähte davon eine größere Dicke aufweisen. Die dickeren Längsdrähte sind vorzugsweise gleichmäßig über den Umfang des Gitterkörpers verteilt, beispielsweise bei vier dickeren Längsdrähten jeweils um einen Viertelumfang zueinander versetzt.

Bei einer weiteren Ausführungsform sind als stabilisierende Verstrebungen Längsröhrchen vorgesehen. Insbesondere kann der Gitterkörper ein Drahtgitter aufweisen, in dem die Längsröhrchen wie Längsdrähte angeordnet sind. Auf diese Weise kann die Steifigkeit des Gitterkörpers erhöht werden.

In Kombination der Messeinrichtung mit der Einspeiseeinrichtung kann es sich bei dem zumindest einem Sensor oder einem von mehreren Sensoren um einen solchen handeln, mittels dem der Zustand und/oder der Betrieb der Einspeiseeinrichtung überwacht werden kann. Bei dem Sensor oder einem von mehreren Sensoren kann es sich beispielsweise um einen Verschleißsensor handeln, der den Verschleiß einer Komponente des Anströmkörpers überwacht, zum Beispiel des Gitterkörpers. Auf diese Weise kann die Einspeiseeinrichtung rechtzeitig vor deren Versagen ausgetauscht werden. Bei dem oder einem Sensor kann es sich weiterhin um einen Vibrationssensor handeln, der die Vibration des Anströmkörpers im Gasstrom misst. Auf diese Weise kann zum Beispiel eine Versagensprognose erfolgen. Bei dem oder einem Sensor kann es sich weiterhin um einen Versagenssensor, beispielsweise um einen Bruchsensor handeln, der das Versagen einer Komponente des Anströmkörpers, zum Beispiel des Gitterkörpers, überwacht. Auf diese Weise kann die Einspeiseeinrichtung zeitnah nach einem Versagen, zum Beispiel durch einen Druckstoß in der Gasleitung, ausgetauscht werden. Der Verschleiß- oder Versagenssensor kann insbesondere an oder in einer der stabilisierenden Verstrebungen angeordnet werden. Bei dem oder einem Sensor kann es sich weiterhin um einen Strömungsgeschwindigkeitssensor handeln, der die Strömungsgeschwindigkeit des Gasstroms in der Gasleitung misst. Auf diese Weise kann zum Beispiel eine Regelung der auf den Verdunstungskörper gegebenen Odoriermittelmenge abhängig von der Strömungsgeschwindigkeit des Gasstroms erfolgen.

Außerdem kann die Messeinrichtung eine externe Auswerte- oder Überwachungselektronik aufweisen, die mit der Empfangseinheit verbunden oder verbindbar ist. Auf diese Weise wird zum Beispiel ein Anschluss des Sensors an einen lokale Auswerte- oder Überwachungselektronik oder, durch dazwischen schaltbare Datenfernübertragungsmittel, an eine ferne Auswerte- oder Überwachungselektronik ermöglicht. Auf diese Weise kann zum Beispiel eine zentrale Überwachung verschiedener Messeinrichtungen und/oder Einspeiseeinrichtungen ermöglicht werden, so dass zum Beispiel bei einem Versagen eine schnelle Instandsetzung erfolgen kann. Weiterhin können die mit dem zumindest einen Sensor gewonnenen Daten auch zur Überwachung z. B. des Gasleitungsnetzes, der Gaszusammensetzung, dem Odoriermittelanteil im Gasstrom, die Vollständigkeit der Verdampfung des Odoriermittels oder dem Zustand der Einspeiseeinrichtung verwendet werden.

Möglich ist auch, dass der Anströmkörper halbzylinderförmig ausgebildet ist, wobei der gerundete Teil des Anströmkörpers insbesondere in die Strömung gestellt wird. Es wurde festgestellt, dass es bei einer Halbzylinderform des Anströmkörpers auf der flachen Rückseite zu Verwirbelungen des Gasstroms kommt, wodurch eine höhere Verdunstungsleistung erzielt wird. Ein solcher halbzylinderförmige Anströmkörper lässt sich insbesondere im 3D-Druck herstellen.

Gemäß einem zweiten Aspekt der Erfindung wird die eingangs erwähnte Aufgabe gelöst durch eine Vorrichtung mit einer Messeinrichtung nach dem ersten Aspekt der Erfindung und einer Einsatzeinheit, die ein Gehäuse mit einem gasdurchströmbaren Gasleitungsabschnitt zum Einbau in eine Gasleitung aufweist, wobei die Messeinrichtung derart in eine Öffnung in einer Wand des Gehäuses eingesetzt oder einsetzbar ist, dass sich der Anströmkörper in den Gasleitungsabschnitt erstreckt.

Bei Vorsehen einer Einspeiseeinrichtung in der Messeinrichtung, wie zuvor beschrieben worden ist, kann demgemäß eine Vorrichtung zur Einspeisung eines flüssigen Odoriermittels bereitgestellt werden, welche ein Gehäuse mit einem sich darin erstreckenden Verdunstungskörper umfasst, in welchen über eine Odoriermitteldüse ein flüssiges Odoriermittel so eingebracht werden kann, dass der Verdunstungskörper benetzt wird und die Verdunstung des Odoriermittels fördert.

Es kann vorgesehen sein, dass die Einsatzeinheit einen Ventilkörper aufweist, der von einer ersten geöffneten Stellung in eine zweite geschlossene Stellung verbringbar ist, wobei der Ventilkörper in der zweiten Stellung den Gasleitungsabschnitt verschließt und in der ersten Stellung freigibt und wobei der Ventilkörper in der zweiten Stellung mit dem Gehäuse eine Entnahmeschleuse für die Messeinrichtung bildet. Dies ermöglicht eine einfache Entnahme der Messeinrichtung und damit einen einfachen Austausch der Messeinrichtung.

Demnach kann das Gehäuse gleichzeitig als Entnahmeschleuse für die Messeinrichtung, insbesondere den Anstromkörper und optional für die Einspeiseeinrichtung und Odoriermitteldüse und auch den Verdunstungskörper, ausgebildet sein. Der Anstromkörper kann sich im Wesentlichen über die gesamte lichte Weite des freien Gehäusequerschnitts innerhalb des Gehäuses erstrecken.

Möglich ist, dass die Entnahmeschleuse für die Messeinrichtung, insbesondere den Anstromkörper und optional für die Odoriermitteldüse und auch den Verdunstungskörper, durch nur einen einzigen Ventilkörper gebildet wird, der gleichzeitig den Querschnitt der Gasleitung versperrt. Dadurch entfällt die Notwendigkeit, das Gehäuse der Vorrichtung zur Einspeisung des flüssigen Odoriermittels jeweils beiderseits eines Anschlusses an die Gasleitung absperren zu müssen.

Der Ventilkörper kann eine Schleusenkammer aufweisen, die mit einem Ventilsitz des Gehäuses so zusammenwirkt, dass der Ventilsitz die Schleusenkammer abdichtet, wenn sich der Ventilkörper in der zweiten, geschlossenen Stellung befindet. Als Schleusenkammer kann beispielsweise ein Hohlraum im Ventilkörper vorgesehen sein, der so bemessen ist, dass dieser den Anstromkörper (optional mit Verdunstungskörper) und optional auch Teile der Odoriermitteldüse aufnehmen kann.

Möglich ist ferner, dass eine Einrichtung zur Vorentspannung der Schleusenkammer vorgesehen ist. Als Vorentspannungseinrichtung zur Vorentspannung der Schleusenkammer kann beispielsweise die Odoriermitteldüse ein Vorentspannungsventil aufweisen, das zweckmäßigerweise an einem Teil der Odoriermitteldüse angeordnet ist, der sich außerhalb des Gehäuses erstreckt. Dadurch ist es möglich, nach dem Absperren des freien Querschnitts des Gehäuses zunächst die Schleusenkammer von dem in dem Gehäuse vorherrschenden Druck zu entlasten, sodass das Gehäuse sodann von außen zwecks Entnahme der Messeinrichtung geöffnet werden kann.

Der Anstromkörper mit dem zumindest einem Sensor, sowie optional der Verdunstungskörper, kann beispielsweise in einer Tauchhülse oder einem Tauchrohr, insbesondere der Odoriermitteldüse, angeordnet sein. Der Anstromkörper mit dem Verdunstungskörper und dem zumindest einen Sensor und die Odoriermitteldüse können beispielsweise als eine ganze zu handhabende Einheit in Form einer Patrone ausgebildet sein.

Bei einer möglichen Ausgestaltung der Vorrichtung ist der Ventilkörper als Kugelventilkörper ausgebildet, der einen Durchströmungskanal aufweist. Der Anstromkörper, mit oder ohne Verdunstungskörper, und/oder die Odoriermitteldüse, können beispielsweise eine Durchführung in einem Pol des Kugelventilkörpers durchsetzen. Ein solcher Kugelventilkörper umfasst zweckmäßigerweise zwei diametral gegenüber liegende Pole, die die Symmetrieachse und Rotationsachse des Kugelventilkörpers definieren. An einem Pol kann eine Schaltwelle zur Betätigung des Kugelventilkörpers angreifen und an dem gegenüberliegenden Pol kann eine Durchführung vorgesehen sein, in die der Anstromkörper, insbesondere der Verdunstungskörper, und/oder die Odoriermitteldüse eintauchen. Zweckmäßigerweise ist der Kugelventilkörper über ein Kugelhahn betätigbar, der an einer das Gehäuse durchsetzenden Schaltwelle befestigt ist.

Bei einer alternativen Ausgestaltung der Vorrichtung kann der Ventilkörper als Schieber, insbesondere als Keilschieber, ausgebildet sein, der über einen Spindeltrieb betätigbar ist.

In einer weiteren Variante der Vorrichtung kann vorgesehen sein, dass das Gehäuse einen ersten, zweiten und dritten Rohrleitungsabgang aufweist, dass der Ventilkörper in der zweiten geschlossenen Stellung den dritten Rohrleitungsabgang freigibt und dass der dritte Rohrleitungsabgang mit einem Revisionsdeckel verschlossen ist. Bei dieser Variante der Vorrichtung begrenzt der Revisionsdeckel mit dem Gehäuse die Entnahmeschleuse. Der Revisionsdeckel kann beispielsweise mit einem Schauglas versehen sein, das eine Sichtkontrolle der Entnahmeschleuse, des Anstromkörpers oder der Odoriermitteldüse ermöglicht. Der erste, zweite und der dritte Rohrleitungsabgang können alle etwa den gleichen Querschnitt aufweisen, wobei vorzugsweise der erste und der zweite Rohrleitungsabgang den gasdurchströmbaren Gasleitungsabschnitt der Vorrichtung bilden. Durch den dritten Rohrleitungsabgang wird eine verhältnismäßig große Zugangsöffnung zu der Entnahmeschleuse bereitgestellt. Dadurch besteht die Möglichkeit, ein Bauteil, wie beispielsweise die Odoriermitteldüse oder den Anströmkörper, von der Seite in die Vorrichtung einzusetzen. Hierdurch steht ein größerer Querschnitt zum Einbringen von Bauteilen in die Vorrichtung zur Verfügung. Eine Vorentspannung der Schleusenkammer kann in der zuvor beschriebenen Art und Weise oder über einen Anschluss, welcher im Bereich der Entnahmeschleuse angeordnet ist, erfolgen.

Gemäß einem dritten Aspekt der Erfindung wird die eingangs erwähnte Aufgabe gelöst durch eine Verwendung einer Messeinrichtung nach dem ersten Aspekt der Erfindung oder einer Vorrichtung nach dem zweiten Aspekt der Erfindung für zumindest eine Messung in einer Gasleitung.

Wie zuvor bereits beschrieben, können unterschiedliche Messgrößen im Rahmen der Messung durch einen, mehrere, insbesondere verschiedene oder kombinierte Sensoren erfasst werden. Wie zuvor erläutert, kann der Sensor oder können die Sensoren an dem Anstromkörper, insbesondere an dem Gitterkörper, angeordnet sein und/oder in einem becherförmigen Bodenteil des Gitterkörpers angeordnet sein. Mögliche Messgrößen beziehen sich auf den Gasstrom, umfassen also etwa den Druck, die Temperatur, die Gaszusammensetzung und/oder den Odoriermittelgehalt, den Füllstand bzw. die Flüssigkeitsmenge in dem Bodenteil und/oder einen Verschleiß oder ein Versagen des Anstromkörpers, insbesondere des Gitterkörpers.

Anhand der beigefügten Zeichnungen wird die Erfindung gemäß von Ausführungsbeispielen nachfolgend näher erläutert. Sämtliche aus den Ansprüchen, der Beschreibung oder der Figur hervorgehenden Merkmale, einschließlich konstruktiver Einzelheiten, können sowohl für sich als auch in den beliebigen verschiedenen Kombinationen erfindungswesentlich sein. Es zeigen:
- Fig.1: eine schematische Darstellung einer Vorrichtung zur Odorierung eines Gasstroms in einer Gasleitung,
- Fig. 2: ein erstes Ausführungsbeispiel einer Einspeiseeinrichtung,
- Fig. 3: das Ausführungsbeispiel aus Fig. 2 in einer Einbausituation und ein Ausführungsbeispiel einer Vorrichtung,
- Fig. 4a-b: einen Anströmkörper eines weiteren Ausführungsbeispiels einer Einspeiseeinrichtung,
- Fig. 5a-b: einen Anströmkörper eines weiteren Ausführungsbeispiels einer Einspeiseeinrichtung,
- Fig. 6a-6b: einen Anströmkörper eines weiteren Ausführungsbeispiels einer Einspeiseeinrichtung,
- Fig. 7: einen Querschnitt eines Anströmkörpers eines weiteren Ausführungsbeispiels einer Einspeiseeinrichtung,
- Fig. 8a-b: einen Gitterkörper eines Anströmkörpers eines weiteren Ausführungsbeispiels einer Einspeiseeinrichtung,
- Fig. 9a-b: einen Gitterkörper eines Anströmkörpers eines weiteren Ausführungsbeispiels einer Einspeiseeinrichtung,
- Fig. 10: ein Ausführungsbeispiel einer Messeinrichtung, und
- Fig. 11a-b: ein weiteres Ausführungsbeispiel einer Vorrichtung.

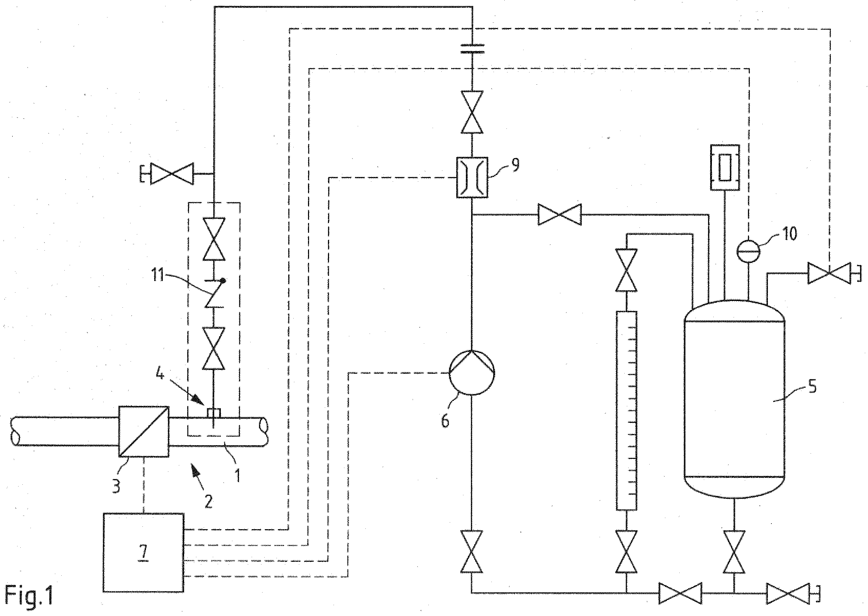

Elemente mit gleicher Funktion und Wirkungsweise sind in den Figuren 1 bis 11 jeweils mit denselben Bezugszeichen versehen.

Figur 1 zeigt zunächst den grundsätzlichen Aufbau einer Vorrichtung zur Odorierung eines Gasstroms in schematischer Darstellung. In Figur 1 ist ein Gasleitungsabschnitt 1 einer Gasleitung 2 dargestellt, der in Strömungsrichtung des Gases einem Gaszähler 3 nachgeschaltet ist. Die Vorrichtung zur Odorierung kann beispielsweise in einer GDRM-Anlage eines örtlichen Gasverteilnetzes angeordnet sein. In der Gasleitung 2 wird vorliegend aus einem Hochdrucktransportnetz Erdgas für das Gasdruckverteilnetz mit einem reduzierten Druck bereitgestellt.

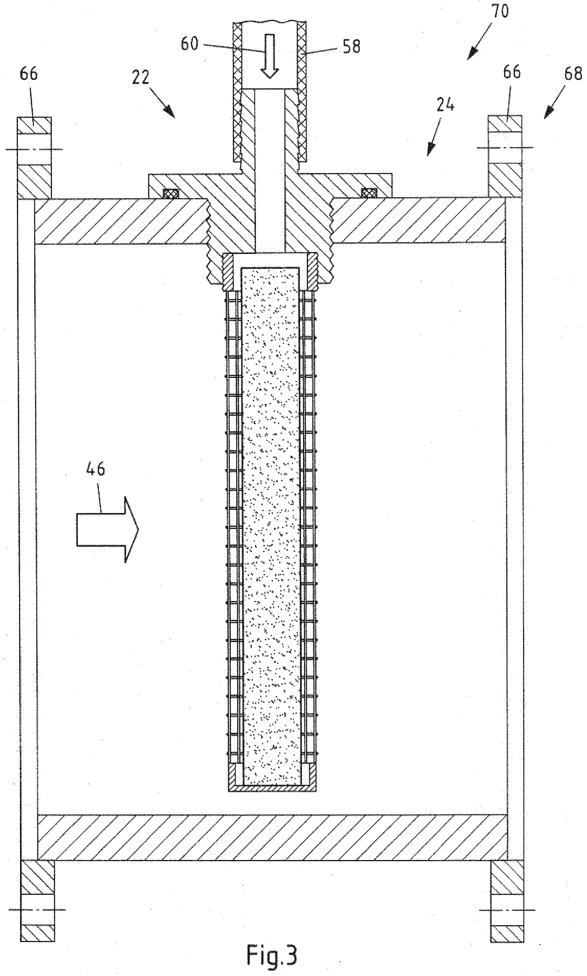

In Strömungsrichtung hinter dem Gaszähler 3 wird ein flüssiges Odoriermittel 60 (siehe Fig. 3), beispielsweise in Form von THT, über eine Einspeiseeinrichtung 4 in die Gasleitung 2 eingebracht. Dazu wird das Odoriermittel einem Odoriermittelgebinde 5 entnommen und der Einspeiseeinrichtung 4 über eine Dosierpumpe 6 zugeführt. Die Dosierpumpe 6 ist über ein Steuergerät 7 mit dem Gaszähler 3 verbunden. Der Gaszähler 3 liefert an das Steuergerät 7 eine Information über den Gasvolumenstrom in der Gasleitung 2 und das Steuergerät 7 steuert die Dosierpumpe 6 derart an, dass die Dosierpumpe 6 eine an den Gasvolumenstrom angepasste Menge Odoriermittel zur Einspeiseeinrichtung 4 fördert. Zur Regelung der Dosierpumpe 6 kann ein Durchflussmesser 9 vorgesehen sein, der den von der Dosierpumpe 6 gepumpten Odoriermittelvolumenfluss misst. Weiterhin kann ein Füllstandmesser 10 vorgesehen sein, um den Füllstand im Odoriermittelgebinde 5 zu überwachen. Zwischen der Dosierpumpe 6 und der Einspeiseeinrichtung 4 ist vorzugsweise eine Rückschlagklappe 11 vorgesehen, um eine Gasströmung aus der Gasleitung 2 zum Odoriermittelgebinde 5 zu verhindern.

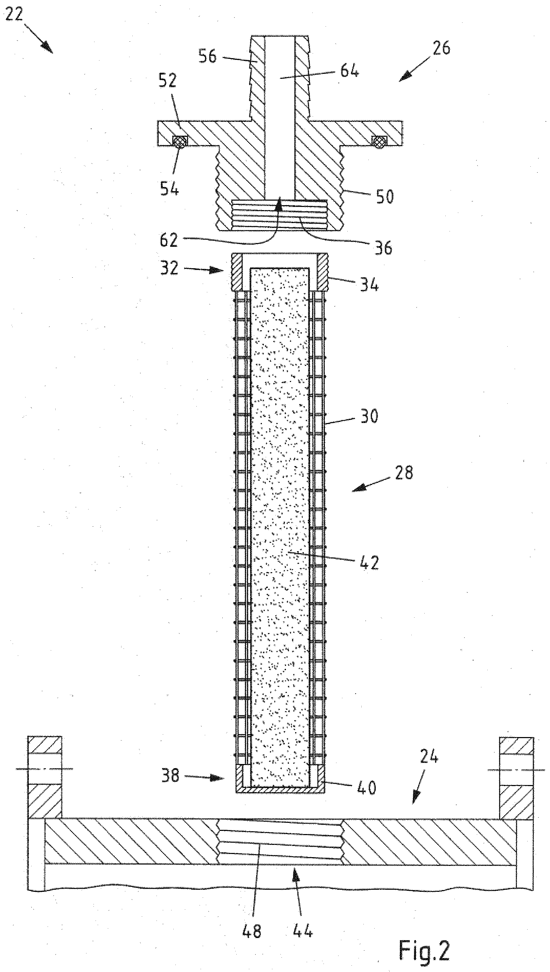

Die Figuren 2 und 3 zeigen nun ein erstes Ausführungsbeispiel einer Einspeiseeinrichtung 22. Fig. 2 zeigt eine Ansicht der Einzelteile der Einspeiseeinrichtung 22 und Fig. 3 die Einbausituation in einem Gasleitungsabschnitt 24. Die Einspeiseeinrichtung 22 weist eine Odoriermitteldüse 26 und einen Anströmkörper 28 auf. Der Anströmkörper 28 weist einen zylinderförmigen Gitterkörper 30 auf, an dessen einem Ende 32 ein Außengewinde 34 vorgesehen ist, mit dem der Gitterkörper 30 in ein dazu komplementäres Innengewinde 36 der Odoriermitteldüse 26 geschraubt werden kann. Alternativ kann der Anströmkörper 28 an dessen einem Ende 32 auch mit einem Innengewinde ausgeführt werden, das auf ein dazu komplementäres Außengewinde der Odoriermitteldüse 26 geschraubt werden kann. An seinem dem Ende 32 gegenüberliegenden Ende 38 weist der Gitterkörper 30 ein becherförmiges Bodenteil 40 auf. In dem Gitterkörper 30 ist ein Verdunstungskörper 42 aus einem offenzelligen Schaum, zum Beispiel aus einem offenzelligen Metall- oder Keramikschaum angeordnet.

Die Odoriermitteldüse 26 ist in Form einer Tauchhülse ausgebildet, so dass sie in eine Wandöffnung 44 des Gasleitungsabschnitts 24 eingeführt werden kann, um den mit der Odoriermitteldüse 26 verschraubten Anströmkörper 28 in einem in dem Gasleitungsabschnitt 24 strömenden Gasstrom 46 zu positionieren. Fig. 3 zeigt die Einspeiseeinrichtung 22 in zusammengesetztem Zustand und nach dem Einbau in den Gasleitungsabschnitt 24.

Die Odoriermitteldüse 26 weist vorzugsweise Befestigungsmittel auf, die ein gasdichtes Einsetzen und Befestigen der Odoriermitteldüse 26 am Gasleitungsabschnitt 24, wie in Fig. 3 gezeigt, erlauben. Bei der Odoriermitteldüse 26 sind hierzu zum Beispiel ein zu einem Innengewinde 48 der Wandöffnung 44 korrespondierendes Außengewinde 50 sowie ein Flansch 52 zur äußeren Anlage an den Gasleitungsabschnitt vorgesehen. Der Flansch kann an seiner Unterseite zum Beispiel einen umlaufenden Dichtring 54 aufweisen.

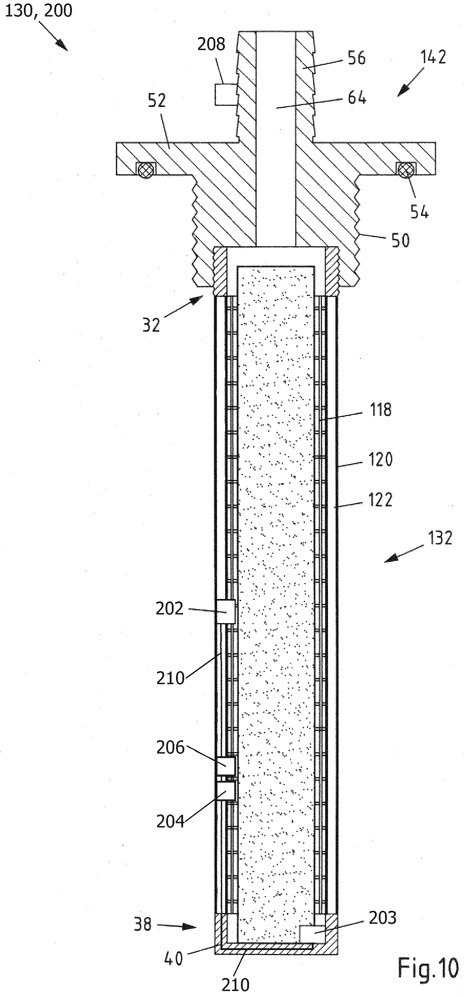

Weiterhin weist die Odoriermitteldüse 26 einen Anschlussstutzen 56 zum Anschluss einer Odoriermittelzuleitung 58 auf, so dass im Betrieb Odoriermittel 60 aus der angeschlossenen Odoriermittelzuleitung 58 durch einen vom Anschlussstutzen 56 durch die Odoriermitteldüse 26 zu einer Öffnung 44 verlaufenden Kanal 122 (siehe Fig. 10) auf den Verdunstungskörper 42 gelangt.

Der Verdunstungskörper 42 hat aufgrund seiner offenzelligen Schwammstruktur eine sehr große innere Oberfläche, über die sich das im Betrieb auf den Verdunstungskörper 42 gelangende Odoriermittel 60 verteilt. Die Porendichte des Verdunstungskörpers 42 ist mit 10-60 ppi zudem für die typischerweise verwendeten Odoriermittel 60 (z. B. Tetrahydrotiophen) und die typischen Gasstromgeschwindigkeiten in Gasleitungen 2 optimiert, so dass mit der Einspeiseeinrichtung 22 eine hohe Verdunstungsleistung erreicht wird.

Der Anströmkörper 28 kann aufgrund seiner hohen Verdunstungsleistung entsprechend sehr kompakt ausgebildet werden und weist eine Länge zwischen 50 und 600 mm und einen Durchmesser im Bereich von 0,5 bis 1 Zoll auf. Auf diese Weise ist die Einspeiseeinrichtung 22 einfacher zu handhaben und kann auch in Gasleitungen 2 mit geringeren Nennweiten eingesetzt werden.

Durch das am unteren Ende 38 des Gitterkörpers 30 vorgesehene becherförmige Bodenteil 40 wird erreicht, dass etwaiges überschüssiges Odoriermittel 60, zum Beispiel bei zu großer Odoriermittelzufuhr oder bei reduziertem Gasstrom 46, nicht auf die Wandung des Gasleitungsabschnitts 24 tropft und diesen verunreinigt, sondern im becherförmigen Bodenteil 40 aufgefangen wird. Der Verdunstungskörper 42 taucht bis zum Grund in das becherförmige Bodenteil 40 ein, so dass das sich ansammelnde Odoriermittel 60 durch Kapillarkräfte wieder in den Verdunstungskörper 42 eingesaugt wird und verdampfen kann.

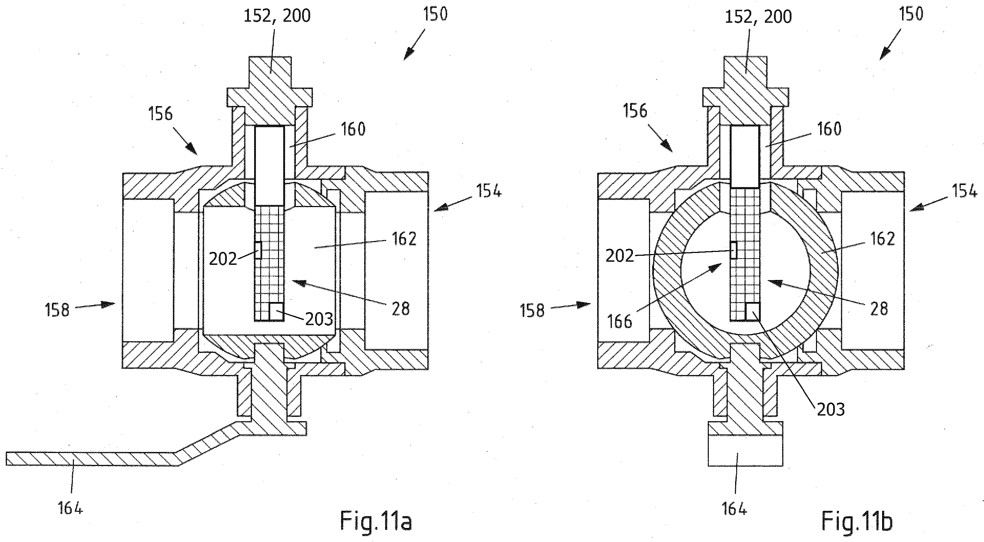

Fig. 3 zeigt die Einbausituation der Einspeiseeinrichtung 22 im Gasleitungsabschnitt 24. Bei dem Gasleitungsabschnitt 24 kann es sich zum Beispiel um eine ggf. längere Gasleitung 2 handeln, um ein kurzes, zum Beispiel mit Verbindungsflanschen 66 versehenes Rohrstück zum Einbau in eine Gasleitung 2 oder um eine Rohrarmatur (s. hierzu auch das Beispiel in Fig. 11). Unabhängig von der konkreten Ausgestaltung stellen der Gasleitungsabschnitt 24 mit der Wandöffnung 44 eine Einsatzeinheit 68 und die Gesamtheit aus Einsatzeinheit 68 und darin eingesetzter Einspeiseeinrichtung 22 eine Vorrichtung 70 zum Einspeisen eines flüssigen Odoriermittels 60 in einen durch eine Gasleitung 2 strömenden Gasstrom 46 dar.

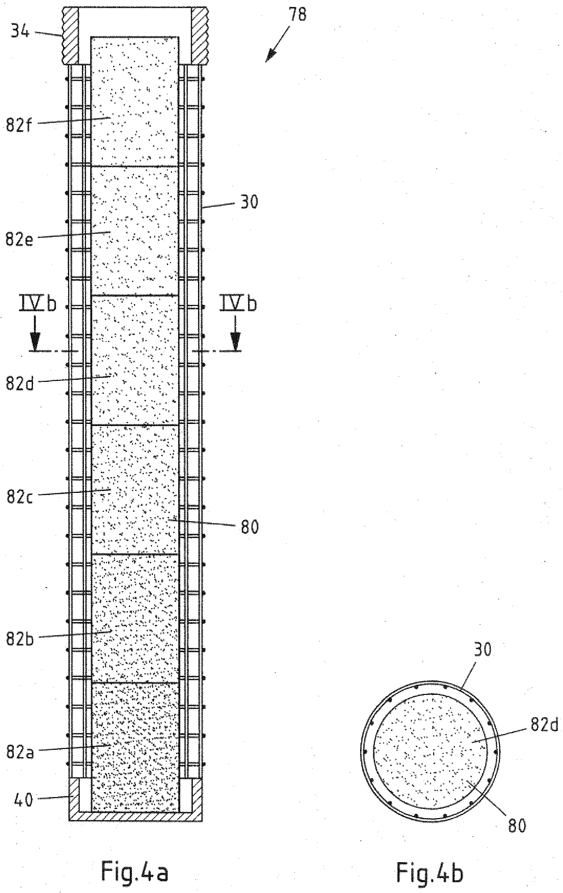

Die Figuren 4a-b zeigen einen Anströmkörper 78 eines weiteren Ausführungsbeispiels einer Einspeiseeinrichtung 22. Fig. 4a zeigt einen Längsschnitt und Fig. 4b zeigt einen Querschnitt entsprechend der in Fig. 4a mit IVb bezeichneten Schnittebene. Der Anströmkörper 78 weist einen ähnlichen Aufbau auf wie der Anströmkörper 28 aus Fig. 2. Einander entsprechende Komponenten sind daher mit gleichen Bezugszeichen versehen und es wird insoweit auf die obige Beschreibung zu Fig. 2 verwiesen. Die Odoriermitteldüse 26 des weiteren Ausführungsbeispiels der Einspeiseeinrichtung 22 weist einen identischen Aufbau auf wie die Odoriermitteldüse 26 aus Fig. 2.

Der Anströmkörper 78 unterscheidet sich dadurch vom Anströmkörper 28 aus Fig. 2, dass der Verdunstungskörper 80 des Anströmkörpers 78 aus einer Mehrzahl übereinander gestapelter Elemente 82a-f aus offenzelligem Schaum mit unterschiedlichen Porendichten und/oder unterschiedlichen mittleren Porengrößen besteht. Auf diese Weise kann berücksichtigt werden, dass die Geschwindigkeit eines Gasstroms in einer Gasleitung über deren Querschnitt nicht konstant ist, sondern eine Geschwindigkeitsverteilung aufweist.

Durch das Stapeln der Elemente 82a-f lassen sich die jeweilige Porendichte und/oder die jeweiligen Porengrößen besser an die verschiedenen Gasgeschwindigkeiten an den jeweiligen Stellen im Gasleitungsquerschnitt anpassen, so dass insgesamt eine bessere Verdunstungsleistung erreicht wird. Zum Beispiel ist die Geschwindigkeit eines Gasstroms 46 an der Wandung der Gasleitung 2 typischerweise geringer als in der Querschnittsmitte der Gasleitung. Die Elemente 82a-f können hierfür beispielsweise so gestapelt werden, dass die (ggf. mittlere) Porendichte und/oder die mittlere Porengröße der einzelnen Elemente vom Element 82a zum Element 82f abnimmt oder zunimmt. Die Elemente 82a-f können insbesondere auch so gestapelt werden, dass die Porendichte und/oder mittlere Porengröße an die jeweilige Position der einzelnen Elemente 82a-f in einer Gasleitung 2 mit vorgegebenem Nenndurchmesser angepasst ist. Bei einer Anordnung entsprechend Fig. 3 könnten die Elemente 82a-f zum Beispiel so angeordnet sein, dass die in diesem Fall in der Mitte der Gasleitung 2 angeordneten Elemente 82c-d die höchste oder niedrigste Porendichte aufweisen und die Porendichte der übrigen Elemente 82a,b,e,f zu den jeweiligen Enden des Anströmkörpers 78 abnimmt bzw. zunimmt.

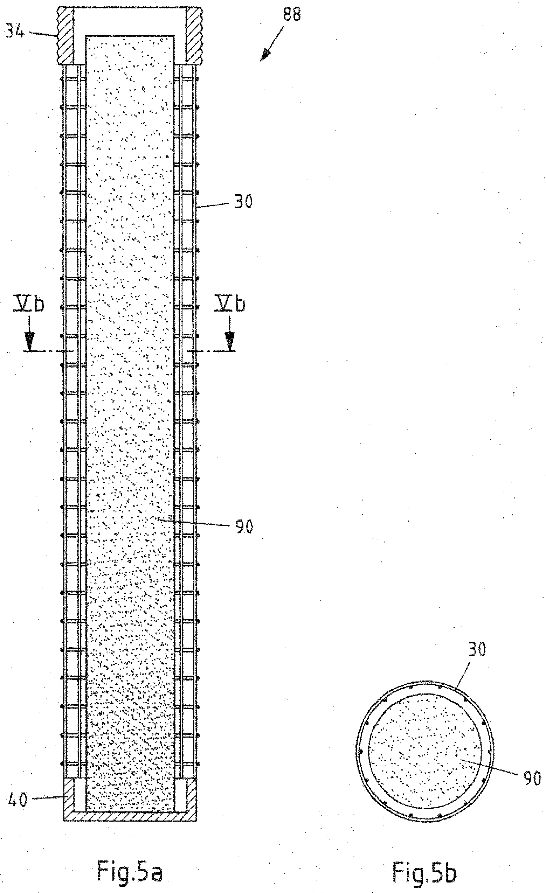

Die Figuren 5a-b zeigen einen Anströmkörper 88 eines weiteren Ausführungsbeispiels einer Einspeiseeinrichtung. Fig. 5a zeigt einen Längsschnitt und Fig. 5b einen Querschnitt entsprechend der in Fig. 5a mit Vb bezeichneten Schnittebene.

Der Anströmkörper 88 weist einen ähnlichen Aufbau auf wie der Anströmkörper 28 aus Fig. 2 bzw. der Anströmkörper 78 aus Fig. 4a-b. Einander entsprechende Komponenten sind daher mit gleichen Bezugszeichen versehen und es wird insoweit auf die obige Beschreibung zu Fig. 2 und 4a-b verwiesen. Die Odoriermitteldüse 26 des weiteren Ausführungsbeispiels der Einspeiseeinrichtung 22 weist einen identischen Aufbau auf wie die Odoriermitteldüse 26 aus Fig. 2.

Der Anströmkörper 88 weist wie der Anströmkörper 78 einen Verdunstungskörper 90 auf, dessen mittlere Porendichte in Längsrichtung des Verdunstungskörpers 90 variiert. Anders als bei dem Verdunstungskörper 80 wird dies jedoch nicht durch übereinanderstapeln einzelner Elemente 82a-f erreicht. Stattdessen ist der Verdunstungskörper 90 in einem Stück gefertigt. Ein solcher einstückiger Verdunstungskörper 90 mit in Längsrichtung variierender Porendichte kann beispielsweise durch 3D-Druck hergestellt werden. Beispielsweise lassen sich durch 3D-Druckverfahren wie selektives Laserschmelzen oder -sintern metallische Schaumstrukturen mit variierender Porendichte herstellen. In entsprechender Weise kann auch ein Anströmkörper 88 mit in Längsrichtung variierender mittlerer Porengröße hergestellt werden.

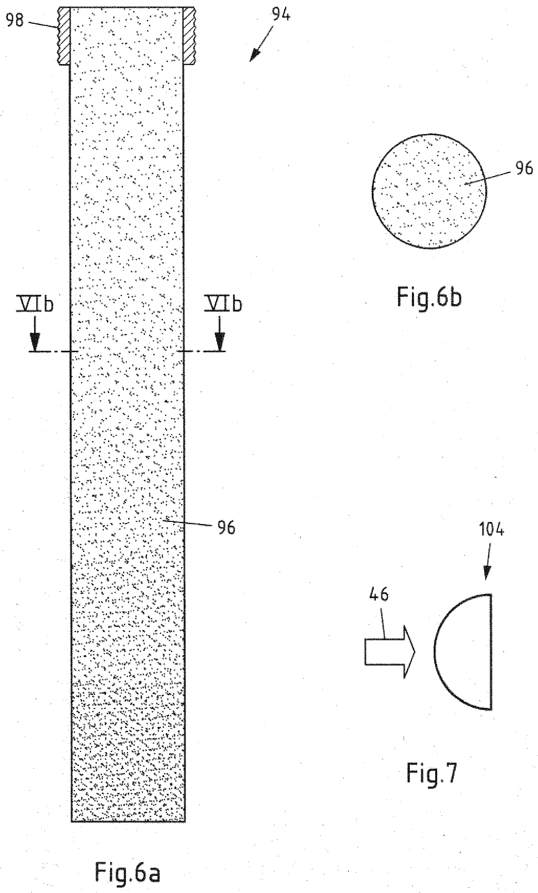

Die Figuren 6a-b zeigen einen Anströmkörper 94 eines weiteren Ausführungsbeispiels einer Einspeiseeinrichtung 22. Fig. 6a zeigt einen Längsschnitt und Fig. 6b zeigt einen Querschnitt entsprechend der in Fig. 6a mit Vlb bezeichneten Schnittebene. Die Odoriermitteldüse 26 dieses Ausführungsbeispiels der Einspeiseeinrichtung 22 weist einen identischen Aufbau auf wie die Odoriermitteldüse 26 aus Fig. 2.

Der Anströmkörper 94 unterscheidet sich dadurch von den Anströmkörpern 28, 78 und 88, dass er keinen separaten Gitterkörper 30 aufweist, sondern durch einen selbsttragenden einstückigen Verdunstungskörper 96 gebildet wird. Ein solcher einstückiger selbsttragender Verdunstungskörper 96 kann im 3D-Druck hergestellt werden, beispielsweise mittels selektivem Laserschmelzen oder -sintern. Insbesondere kann der Verdunstungskörper 96 auch mit einer in Längsrichtung variierender Porendichte und/oder mittlerer Porengröße versehen sein. Vorzugsweise ist der Verdunstungskörper 96 bei der Herstellung im 3D-Druck direkt mit einem Außengewinde 34 zur Befestigung an der Odoriermitteldüse 26 versehen worden, so dass der Verdunstungskörper 96 einfach mit der Odoriermitteldüse 26 verschraubt werden kann.

Fig. 7 zeigt einen Querschnitt eines Anströmkörpers 94 eines weiteren Ausführungsbeispiels einer Einspeiseeinrichtung 22 in schematischer Ansicht. Der Anströmkörper 94 dieses Ausführungsbeispiels kann grundsätzlich einen Aufbau wie der Anströmkörper 28 aus Fig. 2, der Anströmkörper 78 aus Fig. 4a-b, der Anströmkörper 88 aus Fig. 5a-b oder der Anströmkörper 94 aus Fig. 6a-b aufweisen, wobei der Querschnitt 104 des Anströmkörpers - anders als die kreisförmigen Querschnitte der Anströmkörper 28, 78 (s. Fig. 4b), 88 (s. Fig. 5b) und 94 (s. Fig. 6b) - halbkreisförmig ist. Insbesondere können der Gitterkörper 30 und der darin angeordnete Verdunstungskörper 42, 80, 90, 96 des Anströmkörpers 28, 78, 88, 94, 132 oder, insbesondere bei einem selbsttragenden Verdunstungskörper 42, 80, 90, 96 ohne Gitterkörper 30 wie in Fig. 6a-b, nur der Verdunstungskörper 108 einenhalbkreisförmigen Querschnitt 104 aufweisen.

Für den Einsatz wird der Querschnitt 104 des Anströmkörpers 94 so ausgerichtet, dass der gerundete Teil des Querschnitts 104 in Richtung des Gasstroms 46 weist. An den scharfen Kanten des Querschnitts 104 zum flachen Bereich auf der dem Gasstrom 46 abgewandten Seite kommt es so zu Verwirbelungen, die die Verdunstungsleistung des Anströmkörpers 94 verbessern.

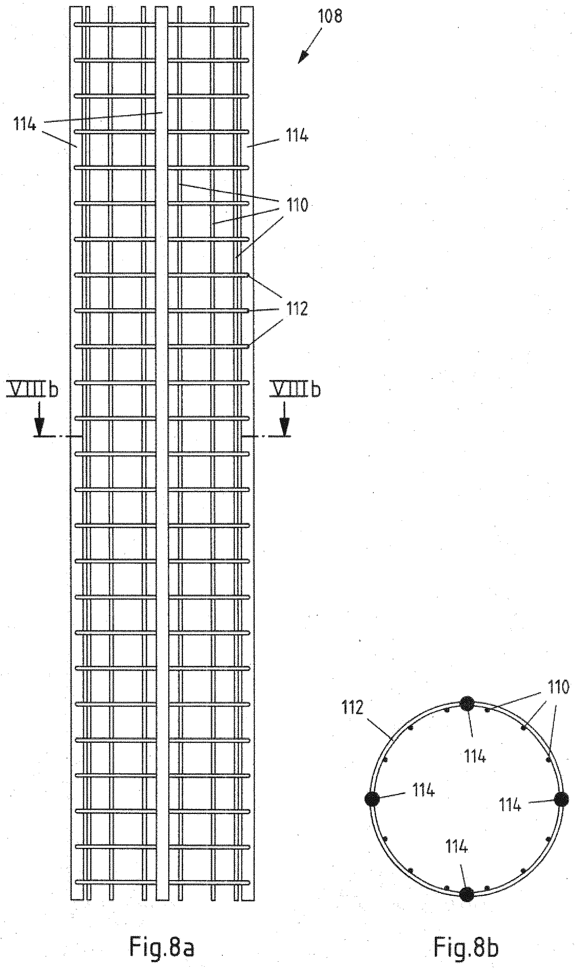

Die Fig. 8a-b zeigen einen Gitterkörper 108 eines Anströmkörpers 28, 78, 88, 94 eines weiteren Ausführungsbeispiels einer Einspeiseeinrichtung 22. Fig. 8a zeigt eine Ansicht von der Seite und Fig. 8b einen Querschnitt entsprechend der in Fig. 8a mit Vlllb bezeichneten Schnittebene. Der Gitterkörper 108 kann beispielsweise anstelle des Gitterkörpers 30 bei einer der zuvor beschriebenen Anströmkörper 28, 78 und 88 eingesetzt werden. Wie der Gitterkörper 30 ist der Gitterkörper 108 am einem Ende mit einem Außengewinde 34 und am anderen Ende vorzugsweise mit einem becherförmigen Bodenteil 40 versehen (in Fig. 8a der Übersicht halber nicht dargestellt).

Der Gitterkörper 108 weist ein Gitter aus dünnen Längsdrähten 110 und Querdrähten 112 auf, die ein Gitter bilden. Vier über den Umfang verteilte Längsdrähte 114 weisen eine größere Dicke auf als die übrigen Längsdrähte 110 und stellen damit stabilisierende Verstrebungen des Gitterkörpers 108 dar. Diese stabilisierenden Verstrebungen stellen auch bei einer geringen Dicke der Längsdrähte 110 eine ausreichende Stabilität des Gitterkörpers 108 sicher, um dem Gasstrom 46 im Gasleitungsabschnitt 24 widerstehen zu können. Die damit ermöglichte geringe Dicke der Längsdrähte 110 und Querdrähte 112 vergrößert den Maschenanteil des Gitterkörpers 108, d. h. den Flächenanteil der Maschen gegenüber der Gesamtfläche inklusive der Längs- und Querdrähte, so dass der Gasstrom mit geringerem Widerstand durch den Gitterkörper 108 strömen kann, wodurch zum einen Verdunstungsleistung erhöht werden kann und zum anderen Vibrationen reduziert werden können.

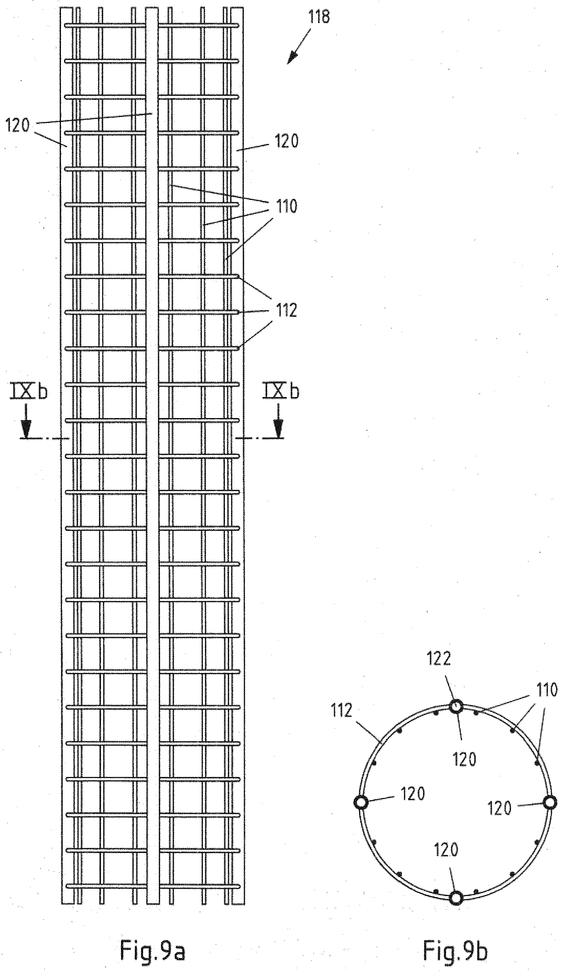

Die Fig. 9a-b zeigen einen Gitterkörper 118 eines Anströmkörpers eines weiteren Ausführungsbeispiels einer Einspeiseeinrichtung 22. Fig. 9a zeigt eine Ansicht von der Seite und Fig. 9b einen Querschnitt entsprechend der in Fig. 9a mit IXb bezeichneten Schnittebene. Der Gitterkörper 118 kann beispielsweise anstelle des Gitterkörpers 30 bei einer der zuvor beschriebenen Anströmkörper 28, 78 und 88 eingesetzt werden. Wie der Gitterkörper 30 ist der Gitterkörper 118 am einem Ende mit einem Außengewinde 34 und am anderen Ende vorzugsweise mit einem becherförmigen Bodenteil 40 versehen (in Fig. 9a der Übersicht halber nicht dargestellt).

Der Gitterkörper 118 weist einen ähnlichen Aufbau auf wie der Gitterkörper 108 aus Fig. 8, wobei einander entsprechende Elemente mit gleichen Bezugszeichen versehen sind und insoweit auf die obige Beschreibung verwiesen wird. Der Gitterkörper 118 unterscheidet sich dadurch vom Gitterkörper 108, dass anstelle der dickeren Längsdrähte 114 Längsröhrchen 120 als stabilisierende Verstrebungen im Gitterkörper 118 angeordnet sind. Die Längsröhrchen 120 erhöhen die Steifigkeit des Gitterkörpers 118. Darüber hinaus können die in den Längsröhrchen 120 verlaufenden Kanäle 122 für Sensoren oder Leitungen verwendet werden.

Fig. 10 zeigt ein weiteres Ausführungsbeispiel einer Einspeiseeinrichtung 22. Die Einspeiseeinrichtung 130 weist einen ähnlichen Aufbau auf wie die Einspeiseeinrichtung 22 aus Fig. 2, wobei einander entsprechende Elemente mit gleichen Bezugszeichen versehen sind und insoweit auf die obige Beschreibung zu Fig. 2 Bezug genommen wird. Die Einspeiseeinrichtung 130 unterscheidet sich dadurch von der Einspeiseeinrichtung 22, dass der Anströmkörper 132 der Einspeiseeinrichtung 130 anstelle des Gitterkörpers 30 den Gitterkörper 118 aus Fig. 9 mit den Längsröhrchen 120 aufweist. Die Längsröhrchen 120 sind in Fig. 9 der Darstellung halber mit übertrieben großem Querschnitt eingezeichnet.

Weiterhin ist der Anströmkörper 132 mit mehreren Sensoren 202, 203, einer drahtlosen Sendeeinheit 206, einer autarken Energieversorgungseinheit 204, einer drahtlosen Empfangseinheit 208 und Leitungen 210 ausgestattet. Die vorgenannten Einheiten bilden gemeinsam eine Messeinrichtung 200, welche vorliegend zudem die Einspeiseeinrichtung 22 mit dem Verdunstungskörper 42, 80, 90, 96 und die Odoriermitteldüse 26 umfasst. Insoweit kann bei der in Fig. 10 gezeigten Einrichtung auch von einer kombinierten Einspeise- und Messeinrichtung, im Folgenden nur Messeinrichtung 200, gesprochen werden. Mittels dieser ist ein Odorieren und Messen möglich.

Dabei kann jede der in Fig. 2 bis 9 gezeigten Einspeiseeinrichtungen 4, 22 bzw. Anstromkörper 28, 78, 88, 94 ebenso bzw. auf gleiche Weise mit mehreren oder allen der oben beschriebenen Komponenten 202, 203, 204, 206, 208, 210 der Messeinrichtung 200 ausgestattet sein, damit diese ebenfalls als Messeinrichtungen 200 ausgebildet sind.

Alternativ ist es möglich, die Messeinrichtung 200 separat von der Einspeiseeinrichtung 4, 22, 130 auszubilden, was vorliegend graphisch nicht gezeigt ist. Dazu kann bei der Messeinrichtung 200 aus Fig. 10 auf den Verdunstungskörper 42, 80, 90, 96 und die Odoriermitteldüse 26 verzichtet werden. In diesem Falle kann jeweils separat durch eine Einspeiseeinrichtung 4, 22, 130 in der Gasleitung 2 odoriert und durch eine Messeinrichtung 200 in der Gasleitung 2 gemessen werden.

Bei dem im becherförmigen Bodenteil 40 angeordneten Sensor 203 kann es sich vorliegend beispielsweise um einen Flüssigkeitssensor bzw. Füllstandsensor handeln, mit dem festgestellt werden kann, ob sich Odoriermittel im becherförmigen Bodenteil 40 ansammelt. Über einen solchen Sensor 203 kann beispielsweise die Odoriermittelzufuhr geregelt werden.

Der optional weitere oder alternative Sensor 202 ist zum Messen eines Drucks, einer Temperatur, einer Gaszusammensetzung und/oder eines Odoriermittelgehalts in dem Gasstrom 46 eingerichtet. Möglich ist auch, dass weitere Sensoren 202 (nicht gezeigt) hierzu eingerichtet und an dem Gitterkörper 118 angeordnet sind. Möglich ist beispielsweise, dass ein solcher weiterer Sensor 202 an oder in einem der Längsröhrchen 120 angeordnet ist und zum Beispiel als Vibrationssensor oder auch als Bruchsensor ausgestaltet ist. Auf diese Weise kann eine Überwachung der Einspeiseeinrichtung 130 auf mechanische Belastungen bzw. Versagen erfolgen, so dass bei drohendem oder erfolgtem Versagen ein kurzfristiger Austausch erfolgen kann.

Der mit den Sensoren 202, 203 ausgestattete Anstromkörper 132 bzw. Gitterkörper 118 weist ferner die autarke Energieversorgungseinheit 204 auf, die beispielsweise als eine Generatoreinheit, eine Batterie oder eine Kombination der beiden ausgebildet sein kann. Die autarke Energieversorgungseinheit 204 stellt die Energie- bzw. Stromversorgung der Sensoren 202, 203 sicher. Dazu ist die autarke Energieversorgungseinheit 204 mittels der Leitungen 210 mit den Sensoren 202, 203 verbunden. Die Leitungen 210 verlaufen vorliegend beispielhaft durch einen Kanal 122 eines jeweiligen der Längsröhrchen 120. Ferner sind die Sensoren 202, 203 mittels der Leitungen 210 oder, alternativ, anderen Leitungen (nicht gezeigt) mit der drahtlosen Sendeeinheit 206 verbunden. Die zur drahtlosen Sendeeinheit 206 korrespondierende drahtlose Empfangseinheit 208 ist außerhalb der Gasleitung 2, insbesondere der Einsatzeinheit 68, vorliegend beispielhaft an dem Anschlussstutzen 56 angeordnet.

Die drahtlose Sendeeinheit 206 und die drahtlose Empfangseinheit 208 bilden eine drahtlose Übertragungseinheit der Messeinrichtung 200, welche zur Übertragung der Messdaten der Sensoren 202, 203 nach außerhalb der Gasleitung 2 bzw. der Einsatzeinheit 68 ausgebildet ist. Insgesamt kann die Einsatzeinheit 68 aus einem metallischen Werkstoff ausgebildet sein, sodass diese einen farradayschen Käfig bildet und eine Funkübertragung der Messdaten nicht in Betracht kommt. Eine drahtgebundene Übertragung birgt die Risiken einer möglichen Gasleckage in den jeweiligen Durchführungen und ist in der Konstruktion vergleichsweise komplex. Daher wird eine Übertragung der Messdaten mittels Ultraschalls, Induktion oder Licht bevorzugt, für welche die Sendeeinheit 206 und die Empfangseinheit 208 entsprechend ausgebildet sein können. Über eine weitere, nicht gezeigte Datenfernübertragungseinheit, die mit der Empfangseinheit 208 verbunden sein kann, können die Messdaten per Datenfernübertragung an einen entfernten Rechner zur Auswertung übertragen werden.

Die Figuren 11a-b zeigen ein weiteres Ausführungsbeispiel einer Vorrichtung 150. Die Vorrichtung 150 weist eine Messeinrichtung 200 mit Einspeiseeinrichtung 152 auf, die zum Beispiel wie die in Fig. 2 gezeigte Einspeiseeinrichtung 22 oder eine der anderen zuvor beschriebenen Einspeiseeinrichtungen 4, 22, 130 ausgebildet sein kann und die mit Bezug auf Fig. 10 erläuterten Komponenten der Messeinrichtung 200 aufweisen kann, wobei der Übersichtlichkeit halber nur die Sensoren 202, 203 in den Fig. 11a-b eingezeichnet sind. Weiterhin weist die Vorrichtung 150 eine Einsatzeinheit 154 auf, die ein Gehäuse 156 mit einem gasdurchströmbaren Gasleitungsabschnitt 158 aufweist. Die Einspeiseeinrichtung 152 ist in eine Öffnung 160 in einer Wand des Gehäuses 156 eingesetzt, so dass sich der Anströmkörper 28 in den Gasleitungsabschnitt 158 erstreckt.

Die Einsatzeinheit 154 weist zudem einen Ventilkörper 162 auf, der mittels eines Hebels 164 von einer ersten geöffneten Stellung (in Fig. 11a dargestellt) in eine zweite geschlossene Stellung (in Fig.11b dargestellt) verbringbar ist, wobei der Ventilkörper 162 in der zweiten Stellung den Gasleitungsabschnitt 158 verschließt und in der ersten Stellung freigibt und wobei der Ventilkörper 162 in der zweiten Stellung mit dem Gehäuse 156 eine Entnahmeschleuse 166 für die Messeinrichtung 200 bildet. Vorliegend ist der Ventilkörper 162 beispielhaft als ein Kugelventilkörper ausgebildet und der Hebel 164 beispielhaft als ein Kugelhahn ausgebildet.

Die Vorrichtung 150 erlaubt die einfache und schnelle Entnahme bzw. den Austausch der Entnahmeschleuse 166, da mit dem Ventilkörper 162 gleichzeitig der Gasstrom 46 im Gasleitungsabschnitt 158 gesperrt und die Entnahme bzw. der Austausch der Messeinrichtung 200 ermöglicht wird.

Für eine Lichtübertragung der Messdaten mittels der drahtlosen Sendeeinheit 206 kann der Ventilkörper 162 über einen Lichtkanal (nicht gezeigt) verfügen, welcher schräg, insbesondere im Wesentlichen orthogonal, zu der in Fig. 11a sichtbaren Durchgangsbohrung des Ventilkörpers 162 zur Öffnung des Gasleitungsabschnitts 158 ausgebildet ist. Der so gebildete Lichtkanal kann zur Übertragung der Lichtsignale mit den Messdaten der Sensoren 202, 203 aus dem Gasstrom 46 heraus genutzt werden, wenn der Ventilkörper 162 sich in der ersten geöffneten Stellung der Fig. 11a befindet. Ferner kann die Einsatzeinheit 154, insbesondere das Gehäuse 156, ein Schauglas oder Sichtfenster (nicht gezeigt) aufweisen, welches einen Blick auf den Anstromkörper 28, 78, 88, 94, 132 ermöglicht. Der Lichtkanal kann derart eingerichtet sein, dass er durch das Schauglas oder Sichtfenster hindurch zur Empfangseinheit 208 führt. Dadurch kann sowohl eine optische Begutachtung von außen durch das Schauglas ermöglicht werden, als auch eine weitere Bohrung in dem Gehäuse 156 zur Durchführung des Lichtkanals vermieden werden. An dem Ventilkörper 162 kann der Lichtkanal gasdicht verschlossen werden. Der Lichtkanal kann beispielsweise durch eine elastische Verklebung einer Linse, insbesondre aus Glas oder Plexiglas, an dem Ventilkörper 162 gebildet werden.

### Bezugszeichenliste

- 1, 24, 158: Gasleitungsabschnitt
- 2: Gasleitung
- 3: Gaszähler
- 4, 22, 130, 152: Einspeiseeinrichtung
- 5: Odoriermittelgebinde
- 6: Dosierpumpe
- 7: Steuergerät
- 9: Durchflussmesser
- 10: Füllstandmesser
- 11: Rückschlagklappe
- 26, 142: Odoriermitteldüse
- 28, 78, 88, 94, 132: Anstromkörper
- 30, 108, 118: Gitterkörper
- 32, 38: Enden des Gitterkörpers
- 34: Außengewinde des Gitterkörpers
- 36: Innengewinde der Odoriermitteldüse
- 40: Bodenteil
- 42, 80, 90, 96: Verdunstungskörper
- 44, 160: Wandöffnung
- 46: Gasstrom
- 48: Innengewinde des Gasleitungsabschnitts
- 50: Außengewinde der Odoriermitteldüse
- 52: Flansch
- 54: Dichtring
- 56: Anschlussstutzen
- 58: Odoriermittelzuleitung
- 60: Odoriermittel
- 66: Verbindungsflansch
- 68, 154: Einsatzeinheit
- 70, 150: Vorrichtung
- 82a-f: gestapelte Elemente aus offenzelligem Schaum
- 104: Querschnitt
- 110, 114: Längsdraht
- 112: Querdraht
- 120: Längsröhrchen
- 122: Kanal
- 156: Gehäuse
- 162: Ventilkörper
- 164: Hebel
- 166: Entnahmeschleuse
- 200: Messeinrichtung
- 202,203: Sensor
- 204: autarke Energieversorgungseinheit
- 206: drahtlose Sendeeinheit
- 208: drahtlose Empfangseinheit
- 210: Leitung

## Patentansprüche

1. Messeinrichtung (200) für eine von einem Gasstrom (46), welcher mit Odoriermittel (60), Biogas, Synthesegas und/oder Wasserstoff angereichert ist, durchströmten Gasleitung (2), wobei
- die Messeinrichtung (200) einen Anstromkörper (28, 78, 88, 94, 132) zur Positionierung in dem Gasstrom (46) in der Gasleitung (2) aufweist,
- der Anstromkörper (28, 78, 88, 94, 132) mit zumindest einem Sensor (202, 203) ausgestattet ist,
- die Messeinrichtung (200) eine drahtlose Sendeeinheit (206) umfasst, welche zum drahtlosen Senden der Messdaten des zumindest einen Sensors (202, 203) an eine außerhalb der Gasleitung (2) befindliche drahtlose Empfangseinheit (208) eingerichtet ist, wobei die drahtlose Sendeeinheit (206) an oder in dem Anstromkörper (28, 78, 88, 94, 132) angeordnet ist, und
- die Messeinrichtung (200) dazu eingerichtet ist, in eine Wandöffnung (44, 160) eines Gasleitungsabschnitts (1, 24, 158) der Gasleitung (2) eingesetzt zu werden, sodass sich der Anstromkörper (28, 78, 88, 94, 132) in den Gasleitungsabschnitt (1, 24, 158) erstreckt.

2. Messeinrichtung (200) nach Anspruch 1, wobei der zumindest eine Sensor (202, 203) zum Messen eines Drucks, einer Temperatur, einer Gaszusammensetzung und/oder eines Odoriermittelgehalts in dem Gasstrom (46) eingerichtet ist.

3. Messeinrichtung (200) nach Anspruch 1 oder 2, wobei der zumindest eine Sensor (202, 203) zumindest eine autarke Energieversorgungseinheit (204) aufweist.

4. Messeinrichtung (200) nach Anspruch 3, wobei die zumindest eine autarke Energieversorgungseinheit (204) als eine Batterie ausgebildet ist.

5. Messeinrichtung (200) nach Anspruch 3 oder 4, wobei die zumindest eine autarke Energieversorgungseinheit (204) als eine Generatoreinheit ausgebildet ist, welche durch den Gasstrom (46) angetrieben wird.

6. Messeinrichtung (200) nach einem der voranstehenden Ansprüche, wobei die drahtlose Sendeeinheit (206) zum drahtlosen Senden der Messdaten mittels Ultraschalls eingerichtet ist.

7. Messeinrichtung (200) nach einem der voranstehenden Ansprüche, wobei die drahtlose Sendeeinheit (206) zum drahtlosen Senden der Messdaten mittels Induktion eingerichtet ist.

8. Messeinrichtung (200) nach einem der voranstehenden Ansprüche, wobei die drahtlose Sendeeinheit (206) zum drahtlosen Senden der Messdaten mittels Lichts eingerichtet ist.

9. Messeinrichtung (200) nach einem der voranstehenden Ansprüche, wobei der Anstromkörper (28, 78, 88, 94, 132) einen Gitterkörper (30, 108, 118) aufweist, an dem der zumindest eine Sensor (202, 203) angeordnet ist.

10. Messeinrichtung (200) nach einem der voranstehenden Ansprüche, wobei die Messeinrichtung (200) eine Einspeiseeinrichtung (4, 22, 130, 152) zum Einspeisen des flüssigen Odoriermittels (60) in den Gasstrom aufweist, wobei die Einspeiseeinrichtung (4, 22, 130, 152) eine Odoriermitteldüse (26, 142) und einen Verdunstungskörper (42, 80, 90, 96) in dem Anstromkörper (28, 78, 88, 94, 132) aufweist, und wobei die Odoriermitteldüse (26, 142) zur Beaufschlagung des Verdunstungskörpers (42, 80, 90, 96) mit dem flüssigen Odoriermittel (60) ausgebildet ist.

11. Messeinrichtung (200) nach Anspruch 9 und 10, wobei der Verdunstungskörper (42, 80, 90, 96) in dem Gitterkörper (30, 108, 118) angeordnet ist.

12. Messeinrichtung (200) nach Anspruch 11, wobei der Gitterkörper (30, 108, 118) an dem von der Odoriermitteldüse (26, 142) entfernten Ende des Gitterkörpers (30, 108, 118) ein becherförmiges Bodenteil (40) aufweist, in den der Verdunstungskörper (42, 80, 90, 96) vorzugsweise eintaucht, wobei der zumindest eine Sensor (202, 203) in dem becherförmigen Bodenteil (40) angeordnet ist.

13. Vorrichtung (70, 150) mit einer Messeinrichtung (200) nach einem der voranstehenden Ansprüche und einer Einsatzeinheit (154), die ein Gehäuse (156) mit einem gasdurchströmbaren Gasleitungsabschnitt (158) zum Einbau in eine Gasleitung aufweist, wobei die Messeinrichtung (200) derart in eine Öffnung (160) in einer Wand des Gehäuses (156) eingesetzt oder einsetzbar ist, dass sich der Anströmkörper (28, 78, 88, 94, 132) in den Gasleitungsabschnitt (158) erstreckt.

14. Vorrichtung (70, 150) nach Anspruch 13, wobei die Einsatzeinheit (154) einen Ventilkörper (162) aufweist, der von einer ersten geöffneten Stellung in eine zweite geschlossene Stellung verbringbar ist, wobei der Ventilkörper (162) in der zweiten Stellung den Gasleitungsabschnitt (158) verschließt und in der ersten Stellung freigibt und wobei der Ventilkörper (162) in der zweiten Stellung mit dem Gehäuse (156) eine Entnahmeschleuse (166) für die Messeinrichtung (200) bildet.

15. Verwendung einer Messeinrichtung (200) nach einem der Ansprüche 1 bis 12 oder einer Vorrichtung (70, 150) nach Anspruch 13 oder 14 für zumindest eine Messung in einer Gasleitung (2).

## Claims

1. Measuring device (200) for a gas line (2) through which a gas stream (46) enriched with odorizing agent (60), biogas, synthesis gas and/or hydrogen flows, wherein
- the measuring device (200) has an inflow body (28, 78, 88, 94, 132) for positioning in the gas stream (46) in the gas line (2),
- the inflow body (28, 78, 88, 94, 132) is equipped with at least one sensor (202, 203),
- the measuring device (200) comprises a wireless transmitting unit (206) which is set up for wireless transmission of the measured data of the at least one sensor (202, 203) to a wireless receiving unit (208) located outside the gas line (2), the wireless transmitting unit (206) being arranged on or in the inflow body (28, 78, 88, 94, 132), and
- the measuring device (200) is set up to be inserted into a wall opening (44, 160) of a gas line section (1, 24, 158) of the gas line (2), so that the inflow body (28, 78, 88, 94, 132) extends into the gas line section (1, 24, 158).

2. Measuring device (200) according to claim 1, wherein the at least one sensor (202, 203) is adapted to measure a pressure, a temperature, a gas composition and/or an odorant content in the gas stream (46).

3. Measuring device (200) according to claim 1 or 2, wherein the at least one sensor (202, 203) comprises at least one self-sufficient energy supply unit (204).

4. Measuring device (200) according to claim 3, wherein the at least one self-sufficient energy supply unit (204) is designed as a battery.

5. Measuring device (200) according to claim 3 or 4, wherein the at least one self-sufficient energy supply unit (204) is designed as a generator unit which is driven by the gas stream (46).

6. Measuring device (200) according to one of the preceding claims, wherein the wireless transmission unit (206) is set up for wireless transmission of the measurement data by means of ultrasound.

7. Measuring device (200) according to one of the preceding claims, wherein the wireless transmission unit (206) is set up for wireless transmission of the measurement data by means of induction.

8. Measuring device (200) according to one of the preceding claims, wherein the wireless transmission unit (206) is set up for wireless transmission of the measurement data by means of light.

9. Measuring device (200) according to one of the preceding claims, wherein the inflow body (28, 78, 88, 94, 132) has a grid body (30, 108, 118) on which the at least one sensor (202, 203) is arranged.

10. Measuring device (200) according to one of the preceding claims, wherein the measuring device (200) comprises a feed device (4, 22, 130, 152) for feeding the liquid odorizing agent (60) into the gas stream, wherein the feed device (4, 22, 130, 152) comprises an odorizing nozzle (26, 142) and an evaporation body (42, 80, 90, 96) in the inflow body (28, 78, 88, 94, 132), and wherein the odorant nozzle (26, 142) comprises an evaporation body (42, 80, 90, 96) in the inflow body (28, 78, 88, 94, 132), 142) and an evaporating body (42, 80, 90, 96) in the inflow body (28, 78, 88, 94, 132), and wherein the odorizing nozzle (26, 142) is designed to act on the evaporating body (42, 80, 90, 96) with the liquid odorizing agent (60).

11. Measuring device (200) according to claims 9 and 10, wherein the evaporation body (42, 80, 90, 96) is arranged in the grid body (30, 108, 118).

12. Measuring device (200) according to claim 11, wherein the grid body (30, 108, 118) at the end of the grid body (30, 108, 118) remote from the odorizing nozzle (26, 142) has a cup-shaped bottom part (40) into which the evaporation body (42, 80, 90, 96) is preferably immersed, wherein the at least one sensor (202, 203) cup-shaped base part (40), into which the evaporation body (42, 80, 90, 96) preferably dips, wherein the at least one sensor (202, 203) is arranged in the cup-shaped base part (40).

13. Device (70, 150) having a measuring device (200) according to one of the preceding claims and an insert unit (154) which has a housing (156) with a gas line section (158) through which gas can flow for installation in a gas line, wherein the measuring device (200) is inserted or can be inserted into an opening (160) in a wall of the housing (156) in such a way that the inflow body (28, 78, 88, 94, 132) extends into the gas line section (158).

14. Device (70, 150) according to claim 13, wherein the insert unit (154) has a valve body (162) which can be moved from a first open position into a second closed position, wherein the valve body (162) closes the gas line section (158) in the second position and releases it in the first position, and wherein the valve body (162) forms an extraction lock (166) for the measuring device (200) with the housing (156) in the second position.

15. Use of a measuring device (200) according to one of claims 1 to 12 or a device (70, 150) according to claim 13 or 14 for at least one measurement in a gas line (2).

## Revendications

1. Dispositif de mesure (200) pour une conduite de gaz (2) traversée par un flux de gaz (46) qui est enrichi en agent odorant (60), en biogaz, en gaz de synthèse et/ou en hydrogène, dans lequel
- le dispositif de mesure (200) présente un corps d'afflux (28, 78, 88, 94, 132) pour le positionnement dans le flux de gaz (46) dans la conduite de gaz (2),
- le corps d'afflux (28, 78, 88, 94, 132) est équipé d'au moins un capteur (202, 203),
- le dispositif de mesure (200) comprend une unité d'émission sans fil (206) qui est conçue pour l'émission sans fil des données de mesure du au moins un capteur (202, 203) vers une unité de réception sans fil (208) située à l'extérieur de la conduite de gaz (2), l'unité d'émission sans fil (206) étant disposée sur ou dans le corps d'afflux (28, 78, 88, 94, 132), et
- le dispositif de mesure (200) est adapté pour être inséré dans une ouverture de paroi (44, 160) d'une section de conduite de gaz (1, 24, 158) de la conduite de gaz (2), de sorte que le corps d'afflux (28, 78, 88, 94, 132) s'étend dans la section de conduite de gaz (1, 24, 158).

2. Dispositif de mesure (200) selon la revendication 1, dans lequel ledit au moins un capteur (202, 203) est adapté pour mesurer une pression, une température, une composition de gaz et/ou une teneur en odorant dans le flux de gaz (46).

3. Dispositif de mesure (200) selon la revendication 1 ou 2, dans lequel ledit au moins un capteur (202, 203) comprend au moins une unité d'alimentation en énergie autonome (204).

4. Dispositif de mesure (200) selon la revendication 3, dans lequel l'au moins une unité d'alimentation en énergie autonome (204) est réalisée sous la forme d'une batterie.

5. Dispositif de mesure (200) selon la revendication 3 ou 4, dans lequel l'au moins une unité d'alimentation en énergie autonome (204) est réalisée sous la forme d'une unité de générateur qui est entraînée par le flux de gaz (46).

6. Dispositif de mesure (200) selon l'une des revendications précédentes, dans lequel l'unité d'émission sans fil (206) est adaptée pour l'émission sans fil des données de mesure au moyen d'ultrasons.

7. Dispositif de mesure (200) selon l'une des revendications précédentes, dans lequel l'unité d'émission sans fil (206) est adaptée pour émettre sans fil les données de mesure par induction.

8. Dispositif de mesure (200) selon l'une des revendications précédentes, dans lequel l'unité d'émission sans fil (206) est adaptée pour l'émission sans fil des données de mesure au moyen de la lumière.

9. Dispositif de mesure (200) selon l'une des revendications précédentes, dans lequel le corps d'afflux (28, 78, 88, 94, 132) présente un corps de grille (30, 108, 118) sur lequel est disposé le au moins un capteur (202, 203).

10. Dispositif de mesure (200) selon l'une des revendications précédentes, le dispositif de mesure (200) présentant un dispositif d'alimentation (4, 22, 130, 152) pour alimenter l'odorant liquide (60) dans le flux de gaz, le dispositif d'alimentation (4, 22, 130, 152) présentant une buse d'agent odorant (26, 142) et un corps d'évaporation (42, 80, 90, 96) dans le corps d'alimentation (28, 78, 88, 94, 132), et la buse d'agent odorant (26, 142) étant conçue pour alimenter le corps d'évaporation (42, 80, 90, 96) avec l'agent odorant liquide (60).

11. Dispositif de mesure (200) selon les revendications 9 et 10, dans lequel le corps d'évaporation (42, 80, 90, 96) est disposé dans le corps de grille (30, 108, 118).

12. Dispositif de mesure (200) selon la revendication 11, dans lequel le corps de grille (30, 108, 118) comporte, à l'extrémité du corps de grille (30, 108, 118) éloignée de la buse d'agent odorant (26, 142), une partie supérieure (30, 108, 118) qui est placée dans le godet en forme de godet (40) dans lequel le corps d'évaporation (42, 80, 90, 96) est de préférence immergé, ledit au moins un capteur (202, 203) étant disposé dans la partie de fond en forme de godet (40).

13. Dispositif (70, 150) avec un dispositif de mesure (200) selon l'une des revendications précédentes et une unité d'insertion (154) qui présente un boîtier (156) avec une section de conduite de gaz (158) pouvant être traversée par le gaz pour le montage dans une conduite de gaz, le dispositif de mesure (200) étant inséré ou pouvant être inséré dans une ouverture (160) dans une paroi du boîtier (156) de telle sorte que le corps d'afflux (28, 78, 88, 94, 132) s'étend dans la section de conduite de gaz (158).

14. Dispositif (70, 150) selon la revendication 13, dans lequel l'unité d'insertion (154) présente un corps de soupape (162) qui peut être déplacé d'une première position ouverte dans une deuxième position fermée, le corps de soupape (162) fermant le tronçon de conduite de gaz (158) dans la deuxième position et le libérant dans la première position, et le corps de soupape (162) formant dans la deuxième position avec le boîtier (156) un sas de prélèvement (166) pour le dispositif de mesure (200).

15. Utilisation d'un dispositif de mesure (200) selon l'une des revendications 1 à 12 ou d'un dispositif (70, 150) selon la revendication 13 ou 14 pour au moins une mesure dans une conduite de gaz (2).
